# EUROPEAN PATENT SPECIFICATION

(11) **EP 3 039 041 B1**
(45) Date of publication and mention of the grant of the patent: **04.03.2020**
(21) Application number: 14777531.6
(22) Date of filing: 26.08.2014
(51) Int. Cl.: C07K 16/28, A61P 9/10, A61P 37/06

(54) **METHOD FOR THE TREATMENT OF FIBROTIC DISEASE**
VERFAHREN ZUR BEHANDLUNG FIBROTISCHER ERKRANKUNGEN
PROCÉDÉ DE TRAITEMENT D'UNE MALADIE FIBROTIQUE

(30) Priority: 30.08.2013 GB 201315486
(43) Date of publication of application: 06.07.2016
(73) Proprietor: UCB Biopharma SRL, 1070 Brussels (BE)
(72) Inventor: MARSHALL, Diane, Slough Berkshire SL1 3WE (GB)
(74) Representative: UCB Intellectual Property
(86) International application number: PCT/EP2014/068047
(87) International publication number: WO 2015/028454

(56) References cited:
- WO-A1-2009/026303
- WO-A1-2010/012667
- WO-A1-2012/082689
- WO-A1-2012/110360
- WO-A1-2013/119716
- WO-A2-2007/143146
- WO-A2-2008/055013
- WO-A2-2013/057281
- BRITTA MAURER ET AL: "Emerging targeted therapies in scleroderma lung and skin fibrosis", BAILLIERE'S BEST PRACTICE AND RESEARCH. CLINICAL REUMATOLOGY, vol. 25, no. 6, 1 January 2011 (2011-01-01), pages 843-858, XP028446276, ISSN: 1521-6942, DOI: 10.1016/J.BERH.2011.11.007 [retrieved on 2011-11-19]
- LIM A K H ET AL: "Antibody blockade of c-fms suppresses the progression of inflammation and injury in early diabetic nephropathy in obese db/db mice", DIABETOLOGIA ; CLINICAL AND EXPERIMENTAL DIABETES AND METABOLISM, SPRINGER, BERLIN, DE, vol. 52, no. 8, 23 May 2009 (2009-05-23), pages 1669-1679, XP019735325, ISSN: 1432-0428, DOI: 10.1007/S00125-009-1399-3
- BYRNE ADAM J ET AL: "Pulmonary Macrophages: A New Therapeutic Pathway in Fibrosing Lung Disease?", TRENDS IN MOLECULAR MEDICINE, ELSEVIER CURRENT TRENDS, GB, vol. 22, no. 4, 12 March 2016 (2016-03-12) , pages 303-316, XP029490435, ISSN: 1471-4914, DOI: 10.1016/J.MOLMED.2016.02.004

## Description

The present invention relates generally to methods of treating fibrotic disease and more specifically to an anti-CSFl-R antibody for the treatment of fibrotic disease.

Colony stimulating factor 1 (CSF-1), also known as macrophage colony stimulating factor (M-CSF) is a cytokine produced by a variety of cells, including endothelial cells and fibroblasts. CSF-1 is composed of two "monomer" polypeptides, which form a biologically active dimeric CSF-1 protein. CSF-1 exists in at least three mature forms due to alternative RNA splicing, proteolytic processing of protein precursors and post-translational modifications including glycosylation and addition of proteoglycan (see, Cerretti DP et al. 1988, Mol Immunol, 25(8),761; Pixley FJ and Stanley ER, 2004, Trends in Cell Biology, 14(11) 628-38; Douglass, TG et al, 2008, Int Immunopharmacol, 8, 1354-76). The variousforms of CSF-1 protein include two secreted molecules, one that is glycosylated, the other comprised of a longer amino terminal sequence and proteoglycan modification. Another variant is a transmembrane (TM) molecule that is glycosylated but has no proteoglycan moieties. This membrane form can be shed via proteolytic cleavage to release an active, soluble molecule. All forms are produced as precursor polypeptides having a 32 amino acid signal sequence at the amino terminus, a putative transmembrane region of approximately 23 amino acids near the carboxyl terminus and a short cytoplasmic COOH-terminal tail. The precursor peptides are subsequently processed by amino terminal and carboxyl terminal proteolytic cleavages to produce the mature forms of CSF-1 with residues 1-149 being identical and constituting the receptor binding domain. *In vivo,* CSF-1 monomers are glycosylated, and dimerized via disulfide-linkage. CSF-1 belongs to a group of biological agonists that promote the production of blood cells. Specifically, it acts as a growth, differentiation and survival factor for bone marrow progenitor cells of the mononuclear phagocyte lineage. Further, CSF-1 stimulates the survival, proliferation and function of macrophages via a specific receptor on responding cells.

The CSF-1 receptor (CSF-1R) is also referred to as the c-fms gene product or CD115. CSF-1R is a 165kDa type 1 TM glycoprotein belonging to the type III receptor tyrosine kinase family. In addition to CSF-1, the structurally similar but sequence unrelated molecule IL-34 has also been shown to be a ligand for CSF-1R (Lin, et al. 2008, Science 320:807-811). Expression of CSF-1R is restricted mainly to cells of the monocyte-macrophage lineage, both circulating and resident tissue populations, including osteoclasts. In addition, it is expressed in a number of cells of the female reproductive system including oocytes, decidual cells and trophoblasts (Pollard JW and Stanley ER, 1996 Advances in Developmental Biochemistry Vol 4, 1996, Pages 153-193 (Pleiotropic Roles for CSF-1 in Development Defined by the Mouse Mutation Osteopetrotic); Arceci RJ, PNAS 1989, 86(22), 8818-8822 (Temporal Expression and Location of CSF-1 and its Receptor in Female Reproductive Tract are consistent with CSF-1-Regulated Placental Development); Arceci, RJ et al, 1992, 151 (1), 1-8; Dev Biol; Regenstreif LJ and Rossant J, Dev Biol 1989 May; 133(1): 284-94 (Expression of the c-fms-oncogene and of the cytokine, CSF-1, during mouse embryogenesis), Pampfer S et al, Biol Reprod 1992, 46(1), 48-57 (Expression of the CSF-1 receptor (c-fms proto-oncogene product) in the human uterus and placenta; Jokhi PP et al, Lab Invest 1993, 68(3), 308-320 (Expression of the CSF-1 Receptor (c-fms product) by cells at the human uteroplacental interface); Kauma SW et al, J Clin Endocrinol Metab 1991, 73(4), 746-751 (CSF-1 and c-fms expression in human endometrial tissues and placenta during the menstrual cycle and early pregnancy), Byrne J Cell Biol 1981 91(3 Pt 1) 848-53, Hofstetter W et al, Bone 1995, 17, (2), 145-151; Tanaka S et al, 1993, J Clin Invest, 91: 257-63; Weir EC et al, 1993, J Bone Miner Res, 8(12) 1507-18.

Binding of the ligand CSF-1 to the CSF-1 receptor results in the phosphorylation of the receptor on one or more tyrosine residues through the action of its tyrosine kinase domain. This phosphorylation can be detected because antibodies are available that bind to the receptor only after phosphorylation (for example Phospho-M-CSF-Receptor (Tyr546) antibody #3083 from Cell Signaling Technology).

Inhibitors of CSF-1 are already known in the art. WO2012082689 and Meurer et al., 2011, Best Practice & Research Clinical Rheumatology, 25:843-858, for instance propose small molecule inhibitors of CSF-1 for use in the treatment of various diseases such as fibrosis.

Antibodies to CSF-1R are known in the art. Sherr, C.J. et al., Blood 73 (1989) 1786-1793 describes antibodies against CSF-1 R that inhibit the CSF-1 activity (Sherr, C.J. et al., Blood 73 (1989) 1786-1793). WO09/026303 discloses anti-CSF-lR antibodies which bind to human CSF-1R and *in vivo* mouse tumour models using an anti-murine CSF-1R antibody. WO11/123381 discloses anti-CSF-lR antibodies which internalize CSF-1R and have ADCC activity. WO11/123381 discloses *in vivo* mouse tumour models using an anti-murine CSF-1R antibody. WO11/140249 discloses anti-CSF-1R antibodies which block binding of CSF-1 to CSF-1R which are stated to be useful in the treatment of cancer. WO09/112245 discloses an anti-CSF-1R IgG1 antibody which inhibits CSF-1 binding to CSF-1R and is stated to be useful in the treatment of cancer, inflammatory bowel disease and rheumatoid arthritis. WO11/131407 discloses an anti-CSF-lR antibody which inhibits CSF-1 binding to CSF-1R and is stated to be useful in the treatment of bone loss and cancer. WO11/107553 discloses an anti-CSF-1R antibody which inhibits CSF-1 binding to CSF-1R and is stated to be useful in the treatment of bone loss and cancer. WO2013/057281 suggests anti-c-fms antibodies for modulating macrophage activation. Lim et al., 2009, Diabetologia, 52:1669-1679, discloses that an anti-CSF-1R antibody could play a role in the development of type 2 diabetic nephropathy. WO11/070024 discloses anti-CSF-1R antibodies which bind to human CSF-1R fragment delD4.

Anti-CSF-1R antibodies are currently in development for treatment of cancer and Rheumatoid Arthritis.

The term 'fibrotic disease' refers to an aberrant wound healing response wherein excess fibrous connective tissue is formed in an organ or tissue. The deposition and accumulation of excess extracellular matrix (ECM) components such as collagen and fibronectin, results in the hardening and scarring of tissues that ultimately can lead to organ failure.

Wounding results in an immediate coagulation and clotting response with the development of a provisional extracellular matrix (ECM). The initial platelet aggregation and activation helps promote an inflammatory response characterised by vasodilation and an increase in blood vessel permeability, allowing the recruitment of a variety of immune cells including neutrophils, macrophages, eosinophils and lymphocytes to the wound site. Neutrophils and macrophages debride the wound thereby reducing the risk of infection and together with activated lymphocytes secrete a variety of growth factors and cytokines that serve to further amplify the inflammatory response. Molecules such as TGFβ, PDGF and IL-13 activate macrophages and lead to the recruitment, proliferation and activation of fibroblasts at the wound site. Activated fibroblasts or myofibroblasts, are characterised by the expression of α-smooth muscle actin and secrete collagen and other ECM components. The activated fibroblasts, contract the collagen lattice, drawing the edges of the wound to the centre. Epithelial and endothelial cells proliferate and migrate over the temporary matrix to regenerate the damaged tissue which is coupled with resolution of the matrix components, completing the wound repair.

However, persistent tissue insult or injury, or a disregulation of the repair pathway leads to an inappropriate wound response. Excess deposition and hyper cross-linking of the collagen and ECM then occurs, resulting in excessive formation and hardening of scar tissue in place of the normal tissue architecture.

A number of approaches have shown that disease severity is reduced when macrophages are depleted in mouse models of lung fibrosis (Zhang-Hoover J et al, Immunology, 2000, 101(4): 501-511; Gibbons MA et al, Am J Respir Crit Care Med, 2011, 184(5); Murray, LA et al, 2011, Int J Biochem Cell Biol, 43, 154-62).Depletion was achieved either through administration of liposomal clodronate or use of a CD11c-diphtheria toxin receptor transgenic mouse, where monocyte/macrophage cells were ablated via injection of the diphtheria toxin molecule. Similarly, in a model of liver fibrosis (Duffield JS et al, J Clin Invest, 2005, 115(1): 56-65) and renal fibrosis (Lin SL, et al, 2009, J Immunol, 183, 6733-43), macrophage depletion reduced pathological damage and collagen deposition. The *op*/*op* mouse has a mutation in the CSF-1 ligand DNA sequence that results in the expression of a truncated, non-functional protein. These mice manifest an osteopetrotic phenotype and are deficient in macrophages. When utilised in a bleomycin model of lung fibrosis, disease severity is greatly reduced compared to wild type mice (Baran et al, 2007, Am J Respir Crit Care Med, 176, 78-89).

Complexity is added to the role of macrophages as they can be classified into at least two functional phenotypes, M1 and M2 (Mantovani, A et al, 2004, Trends in Immunol, 25(12), 677-86; Mantovani, A et al, 2007, Eur J Immunol, 37, 14-16; Mosser DM and Edwards JP, 2008, Nat Rev Immunol, 8, 958-) where M2 macrophages possess pro-fibrotic characteristics (Lupher, ML and Gallatin, WM, 2006, Adv Immunol, 89, 245-88; Prasse A et al, 2006, Am J Resp Crit Care Med, 173, 781-92; Pechkovsky, DV et al, 2010, Clin Immunol, 137 (1), 89-1-1; Gibbons MA et al, 2011, Am J Resp Crit Care Med, 184, 569-81).

Examples of fibrotic diseases include pulmonary fibrosis, such as idiopathic pulmonary fibrosis (IPF), silicosis,cystic fibrosis, renal fibrosis, liver fibrosis, liver cirrhosis, primary sclerosing cholangitis, primary biliary cirrhosis, endomyocardial fibrosis, mediastinal fibrosis, myelofibrosis, retroperitoneal fibrosis, progressive massive fibrosis, nephrogenic systemic fibrosis, Crohn's disease, keloid, myocardial infarction, systemic sclerosis and arthrofibrosis.

The cause of fibrotic disease can be dependent upon the organ or tissue involved and is unknown in some diseases such as idiopathic pulmonary fibrosis. Liver fibrosis and ultimately cirrhosis results from chronic liver damage sustained through exposure to a variety of factors including environmental and dietary factors or infectious agents. Long-term hepatitis B or C infections can cause liver fibrosis. Sustained over consumption of alcohol or a high fat/sugar diet can also lead to cirrhosis of the liver. Similarly, diabetes can damage and scar the kidneys leading to loss of function. In familial pulmonary fibrosis, family members have an increased susceptibility to fibrosis although the genetic factor(s) responsible are unknown.

IPF is a chronic, progressive disease, of unknown cause and is one of seven idiopathic interstitial pneumonias (American Thoracic Society/European Respiratory Society Multidisciplinary Consensus Classification of the Idiopathic Interstitial Pneumonias, 2002, Am J Respir Crit Care Med, 165:277-304). Repetitive injury to alveolar epithelial cells is believed to play a key role in promoting the fibrotic process that results in the typical histopathological features of IPF (Selman M, 2001, Ann Intern Med, 134(2): 136-51; Gross TJ and Hunninghake GW, 2001). A number of risk factors may cause the epithelial cell injury, particularly inhaled toxins and agents. Smoking has been identified as one such likely factor while exposure to wood dusts, metal dusts, silica or other mineral dusts have been reported to increase the risk of developing IPF (reviewed in Borchers AT et al, 2011 Clinic Rev Allerg Immunol, 40: 117-134); Maher TM, 2012, Clin Chest Med, 33: 69-83). The prognosis for patients with IPF is poor with the median survival range being 2-5 years following diagnosis (Gribbin J, et al, 2006, Thorax, 61: 980-85; King TE Jr, 2001, Am J Respir Care Med, 1664: 1171-81; Fernández Perez ER et al, 2010, Chest, 137: 129-37; Lee HL et al, 2005, Chest, 127:2034-41).

Treatment of fibrotic disease typically includes anti-inflammatory and immunosuppressive agents but these are of little benefit for the patient. The lack of efficacy with these treatments contributed to the reconsideration of IPF and fibrotic disease in general, as an aberrant response to wound healing and not an inflammatory condition. Pirfenidone is a small molecule drug that was approved for use in the treatment of IPF in Japan in 2008 and Europe in 2011 which is likely to work via multiple mechanisms of action that are not completely understood but may work in part through a down-regulation of TGF-β. To date, no targeted therapies have been approved for fibrotic indications.

Therefore, there is currently an unmet medical need for improved treatment of fibrotic disease. For example, for IPF there is a 3 year survival rate of 50% and a 5 year survival rate of only 20% and transplantation is required in about 20% of cases.

Accordingly, it is an object of the present invention to provide a new method of treatment of fibrotic disease.

### Summary of the Disclosure

Surprisingly we have been able to demonstrate that inhibitors of CSF-1R activity are active in the treatment of fibrotic disease. Specifically, we have been able to demonstrate that an anti-CSF-lR antibody that inhibits CSF-1R activity is active in *in vivo* animal models of pulmonary fibrosis.

Accordingly, the present invention provides an inhibitor of CSF-1R activity, for the treatment and/or prophylaxis of pulmonary fibrosis, wherein the inhibitor is an antibody or functionally active fragment thereof, and wherein the antibody or fragment thereof binds to CSF-1R.

In the present application, the term "fibrotic disease" includes diseases that are characterised by an aberrant response to wound healing wherein excess fibrous connective tissue is formed in an organ or tissue. Illustrative fibrotic diseases include but are not limited to include pulmonary fibrosis, such as idiopathic pulmonary fibrosis, silicosis and cystic fibrosis; renal fibrosis including tubular atrophy and interstitial fibrosis, liver fibrosis and liver cirrhosis, primary sclerosing cholangitis, primary biliary cirrhosis, endomyocardial fibrosis, mediastinal fibrosis, myelofibrosis, retroperitoneal fibrosis, progressive massive fibrosis, nephrogenic systemic fibrosis, Crohn's disease, keloid, myocardial infarction, scleroderma, systemic sclerosis and arthrofibrosis.

The term 'CSF-1R activity' as used herein refers to the spectrum of activity understood in the art for CSF-1R, in particular the activity of human CSF-1R and isoforms thereof, for example 1, 2, 3 or all isoforms. For example, binding of ligand to the receptor induces phosphorylation of CSF-1R at specific tyrosine residues (Bourette RP and Rohrschneider LR, 2000, Growth Factors 17: 155-166) and the ensuing cascade of signal transduction events can mediate cell migration, survival, differentiation and proliferation (Suzu S et al, 1997, J Immunol, 159, 1860-7; Yeung Y-G and Stanley ER, 2003, Mol Cell Proteomics, 2, 1143-55; Yu W et al 2008, J Leukoc Biol84(3), 852-63). Expression in transfected cells of mutant CSF-1R receptor molecules comprising phenylalanine residues in place of selected tyrosine residues revealed the association of specific tyrosine residues with cellular outcomes such as survival, proliferation and morphology (Yu et al J Leukoc Biol 2008 Sep 84(3): 852-863). Proteomic approaches and immunoblotting techniques using anti-phosphotyrosine antibodies together with molecule specific antibodies, have identified a number of the intracellular molecules involved in mediating these cell functions following ligand stimulation of the receptor (Yeung Y-G et al, 1998, J Biol Chem. 13, 273(46): 17128-37; Husson H et al, 1997, Oncogene15, 14(19): 2331-8.

In one embodiment the subject is a mammal, for example a human.

An inhibitor of CSF-1R activity is an agent that interferes with, for example reduces/inhibits or blocks the activity of CSF-1R, in particular the activity of CSF-1R in fibrotic disease. Particularly preferred are agents which interfere with the activity of CSF-1R in IPF. Inhibitors may partially or completely inhibit CSF-1R activity. Inhibitors of use include without limitation, inhibitors that are capable of interacting with (e.g. binding to, or recognising) the CSF-1 receptor (CSF-1R) or are capable of inhibiting the interaction between CSF-1R and CSF-1 and/or IL-34. In one embodiment the inhibitor blocks binding of CSF-1 to the receptor CSF-1R.

Examples, of suitable inhibitors include an antibody or a fragment thereof that binds to the CSF-1 receptor and interferes with CSF-1 receptor-ligand interaction.

Inhibitors of CSF-1 receptor activity are known in the art as are methods of identifying and producing such inhibitors. Neutralising anti-CSF-1 antibodies have been described, for example by Weir et al., 1996, J Bone Miner. Res.1 1, 1474-1481 and Haran-Ghera et al, 1997, Blood, 89, 2537-2545, which also describes anti-CSF-lR antibodies. Antisense antagonists of CSF-1 have also been described (EP1223980).

In one embodiment, the inhibitor for use in the treatment and/or prophylaxis of fibrotic disease is an antibody or a fragment thereof that inhibits the activity of CSF-1R. Accordingly, there is disclosed the use of an antibody or a fragment thereof that inhibits the activity of CSF-1R for the manufacture of a medicament for use in the treatment and/or prophylaxis of fibrotic disease. Also disclosed is a method of treatment and or prophylaxis of fibrotic disease in a subject comprising administering to said subject a therapeutically effective amount of an antibody or a fragment thereof that inhibits the activity of CSF-1R.

Most preferably, an inhibitor for use in the treatment and/or prophylaxis of fibrotic disease is an antibody or a fragment thereof that interacts with (i.e. binds to or recognises) CSF-1R and inhibits the activity of CSF-1R. In one example the antibodies or fragments thereof selectively interact with CSF-1R. Selectively interacting with (*e.g*. recognising or binding to) means that the antibodies have a greater affinity for CSF-1R polypeptide than for other polypeptides. Examples of suitable antibodies are those that inhibit the activity of CSF-1R by binding to CSF-1R in such a manner as to prevent it being biologically active.

In one embodiment the antibody recognises isoforms of CSF-1R, for example human CSF-1R and isoforms thereof.

In one embodiment the anti-CSF-1R antibody or a fragment thereof is capable of blocking ligand binding to CSF-1R. Blocking as employed herein refers to physically blocking such as occluding the receptor but will also include where the antibody or fragments binds an epitope that causes, for example a conformational change which means that the natural ligand to the receptor no longer binds. Assays suitable for determining the ability of an antibody to block CSF-1R are described in the Examples herein, see Example 2. BIAcore is an example of an assay employed to measure binding kinetics, ELISA assays or cell based assays employing monocytes or THP-1 cells may also be useful. CSF-1 and IL-34 are both ligands for CSF-1R and the anti-CSF-1R antibody may block binding of CSF-1 or IL-34 to CSF-1R but preferably blocks binding of both CSF-1 and IL-34 to CSF-1R. The anti-CSF-lR antibody also preferably does not cause CSF-1R activation or CSF-1R internalisation. Assays suitable for determining the ability of an antibody to cause CSF-1R activation or CSF-1R internalisation are described in the Examples, see Example 2 which describes an assay measuring CSF-1 dependent proliferation.

Accordingly, there is disclosed the use of an anti-CSF-1R antibody for the manufacture of a medicament for use in the treatment and/or prophylaxis of fibrotic disease. Also disclosed is a method of treatment and or prophylaxis of fibrotic disease in a subject comprising administering to said subject a therapeutically effective amount of an anti-CSF-1R antibody.

CSF-1R polypeptides or cells expressing said polypeptides can be used to produce antibodies which specifically recognise said polypeptides. The CSF-1 R polypeptides may be 'mature' polypeptides or biologically active fragments or derivatives thereof.

CSF-1R as employed herein refers to the protein named CSF-1R, isoforms thereof and biologically active fragments thereof. In one embodiment CSF-1R is human protein or an isoform thereof.

Generally an antibody employed in the present invention will be directed to the extracellular domain of CSF-1R.

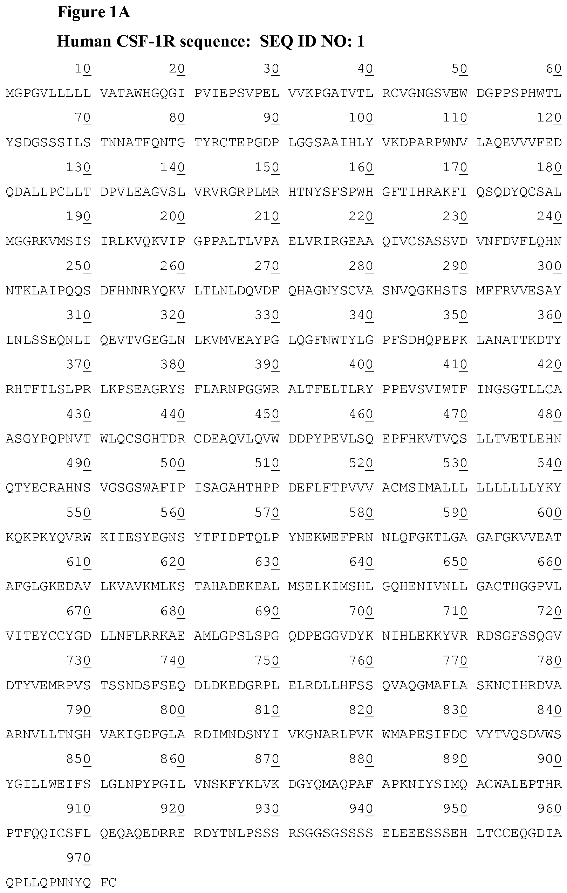

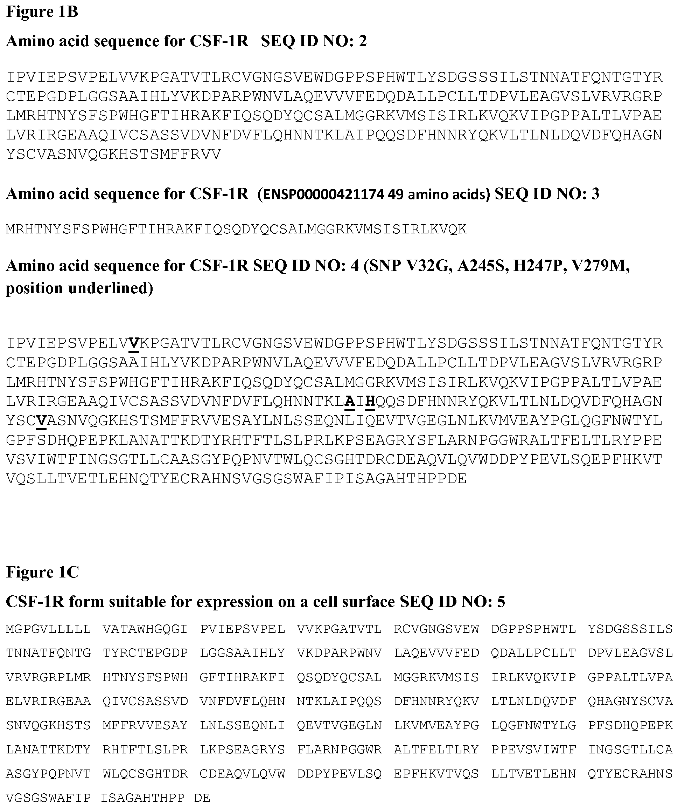

Figure 1A shows the full 972 amino acid sequence of human CSF1-R where residues 1-19 are the predicted signal peptide. Preferably the CSF-1R polypeptide contains amino acids 20-517 of the human CSF-1R sequence shown in Figure 1A. Amino acids 20-517 represent the predicted extracellular region of the CSF-1R sequence. Figure 1B shows some alternative forms of CSF-1R.

Human CSF-1R is registered in the UniProt database under P07333.

CSF-1 and CSF-1 R polypeptides may be prepared by processes well known in the art from genetically engineered host cells comprising expression systems or they may be recovered from natural biological sources.

In one embodiment the sequence shown in Figure 1C may be transfected into a suitable cell line and the polypeptide expressed on the cell surface. The amino acid fragment may be fused to a GPI-anchor to facilitate said expression. The cells may then be employed to immunize hosts.

In the present application, the term "polypeptides" includes peptides, polypeptides and proteins. These are used interchangeably unless otherwise specified. CSF-1 or CSF-1R polypeptides may in some instances be part of a larger protein such as a fusion protein for example fused to an affinity tag.

Antibodies generated against these polypeptides may be obtained by administering the polypeptides or cells expressing the same to an animal, preferably a non-human animal, using well-known and routine protocols, see for example Handbook of Experimental Immunology, D. M. Weir (ed.), Vol 4, Blackwell Scientific Publishers, Oxford, England, 1986. Many warm-blooded animals, such as rabbits, mice, rats, sheep, cows or pigs may be immunized. However, mice, rabbits, pigs and rats are generally preferred.

Monoclonal antibodies may be prepared by any method known in the art such as the hybridoma technique (Kohler and Milstein, 1975, Nature, 256:495-497), the trioma technique, the human B-cell hybridoma technique (Kozbor et al, 1983, Immunology Today, 4:72) and the EBV-hybridoma technique (Cole et al, Monoclonal Antibodies and Cancer Therapy, p77-96, Alan R Liss, Inc., 1985).

Antibodies for use in the invention may also be generated using single lymphocyte antibody methods by cloning and expressing immunoglobulin variable region cDNAs generated from single lymphocytes selected for the production of specific antibodies by for example the methods described by Babcook, J. et al, 1996, Proc. Natl. Acad. Sci. USA 93(15):7843-7848, WO92/02551 and WO2004/051268 and WO2004/106377.

Specific as employed herein is intended to refer to an antibody that only recognises the antigen to which it is specific or an antibody that has significantly higher binding affinity to the antigen to which it is specific compared to binding to antigens to which it is non-specific, for example at least 5, 6, 7, 8, 9, 10 times higher binding affinity.

Chimeric antibodies are those antibodies encoded by immunoglobulin genes that have been genetically engineered so that the light and heavy chain genes are composed of immunoglobulin gene segments belonging to different species. These chimeric antibodies are likely to be less antigenic. Bivalent antibodies may be made by methods known in the art (Milstein et al, 1983, Nature 305:537-539; WO 93/08829, Traunecker et al, 1991, EMBO J. 10:3655-3659). Multi-valent antibodies may comprise multiple specificities or may be monospecific (see for example WO 92/22853).

In one embodiment the antibody for use in the present invention is humanised. As used herein, the term 'humanised antibody molecule' refers to an antibody molecule wherein the heavy and/or light chain contains one or more CDRs (including, if desired, one or more modified CDRs) from a donor antibody (e.g. a murine monoclonal antibody) grafted into a heavy and/or light chain variable region framework of an acceptor antibody (e.g. a human antibody) (see, *e.g.* US 5,585,089; WO91/09967). For a review, see Vaughan et al, Nature Biotechnology, 16, 535-539, 1998.

In one embodiment rather than the entire CDR being transferred, only one or more of the specificity determining residues from any one of the CDRs described herein above are transferred to the human antibody framework (see for example, Kashmiri et al., 2005, Methods, 36, 25-34). In one embodiment only the specificity determining residues from one or more of the CDRs described herein above are transferred to the human antibody framework. In another embodiment only the specificity determining residues from each of the CDRs described herein above are transferred to the human antibody framework.

In a humanised antibody of the present invention, the framework regions need not have exactly the same sequence as those of the acceptor antibody. For instance, unusual residues may be changed to more frequently-occurring residues for that acceptor chain class or type. Alternatively, selected residues in the acceptor framework regions may be changed so that they correspond to the residue found at the same position in the donor antibody (see Reichmann et al., 1998, Nature, 332, 323-324). Such changes should be kept to the minimum necessary to recover the affinity of the donor antibody. A protocol for selecting residues in the acceptor framework regions which may need to be changed is set forth in WO91/09967.

When the CDRs or specificity determining residues are grafted, any appropriate acceptor variable region framework sequence may be used having regard to the class/type of the donor antibody from which the CDRs are derived, including mouse, primate and human framework regions.

Suitably, the humanised antibody has a variable domain comprising human acceptor framework regions as well as one or more of CDRs. Thus, provided in one embodiment is humanised antibody which binds human CSF-1R wherein the variable domain comprises human acceptor framework regions and non-human donor CDRs.

Examples of human frameworks which can be used in the present invention are KOL, NEWM, REI, EU, TUR, TEI, LAY and POM (Kabat *et al., supra*)*.* For example, KOL and NEWM can be used for the heavy chain, REI can be used for the light chain and EU, LAY and POM can be used for both the heavy chain and the light chain. Alternatively, human germline sequences may be used; these are available at: http://vbase.mrc-cpe.cam.ac.uk/
In a humanised antibody of the present invention, the acceptor heavy and light chains do not necessarily need to be derived from the same antibody and may, if desired, comprise composite chains having framework regions derived from different chains.

The antibodies for use in the present invention can also be generated using various phage display methods known in the art and include those disclosed by Brinkman et al. (in J. Immunol. Methods, 1995, 182: 41-50), Ames et al. (J. Immunol. Methods, 1995, 184:177- 186), Kettleborough et al (Eur. J. Immunol. 1994, 24:952-958), Persic et al. (Gene, 1997 187 9-18), Burton et al (Advances in Immunology, 1994, 57:191-280) and WO 90/02809; WO 91/10737; WO 92/01047; WO 92/18619; WO 93/11236; WO 95/15982; WO 95/20401; and US 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108. Techniques for the production of single chain antibodies, such as those described in US 4,946,778 can also be adapted to produce single chain antibodies to CSF-1R polypeptides. Also, transgenic mice, or other organisms, including other mammals, may be used to express humanized antibodies.

The antibody used in the present invention may comprise a complete antibody molecule having full length heavy and light chains or a fragment thereof and may be, but are not limited to Fab, modified Fab, Fab', modified Fab', F(ab')₂, Fv, single domain antibodies (e.g. VH or VL or VHH), scFv, bi, tri or tetra-valent antibodies, Bis-scFv, diabodies, triabodies, tetrabodies and epitope-binding fragments of any of the above (see for example Holliger and Hudson, 2005, Nature Biotech. 23(9):1126-1136; Adair and Lawson, 2005, Drug Design Reviews - Online 2(3), 209-217). The methods for creating and manufacturing these antibody fragments are well known in the art (see for example Verma et al., 1998, Journal of Immunological Methods, 216, 165-181). Other antibody fragments for use in the present invention include the Fab and Fab' fragments described in International patent applications WO2005/003169, WO2005/003170 and WO2005/003171. Multi-valent antibodies may comprise multiple specificities e.g bispecific or may be monospecific (see for example WO 92/22853, WO05/113605, WO2009/040562 and WO2010/035012).

In one embodiment the antibody is provided as CSF-1R binding antibody fusion protein which comprises an immunoglobulin moiety, for example a Fab or Fab' fragment, and one or two single domain antibodies (dAb) linked directly or indirectly thereto, for example as described in WO2009/040562, WO2010/035012, WO2011/030107, WO2011/061492 and WO2011/086091.

In one embodiment the fusion protein comprises two domain antibodies, for example as a variable heavy (VH) and variable light (VL) pairing, optionally linked by a disulphide bond.

In one embodiment the Fab or Fab' element of the fusion protein has the same or similar specificity to the single domain antibody or antibodies. In one embodiment the Fab or Fab' has a different specificity to the single domain antibody or antibodies, that is to say the fusion protein is multivalent. In one embodiment a multivalent fusion protein has an albumin binding site, for example a VH/VL pair therein provides an albumin binding site.

Antibody fragments and methods of producing them are well known in the art, see for example Verma et al, 1998, Journal of Immunological Methods, 216, 165-181. Particular examples of antibody fragments for use in the present invention are Fab' fragments which possess a native or a modified hinge region. A number of modified hinge regions have already been described, for example, in US 5,677,425, WO99/15549, and WO98/25971. Further examples of particular antibody fragments for use in the present invention include those described in international patent applications PCT/GB2004/002810, PCT/GB2004/002870 and PCT/GB2004/002871 (all filed on 1^{st} July 2004). In particular the modified antibody Fab fragments described in International patent application PCT/GB2004/002810 are preferred.

In one embodiment the antibody heavy chain comprises a CH1 domain and the antibody light chain comprises a CL domain, either kappa or lambda.

In one embodiment the antibody heavy chain comprises a CH1 domain, a CH2 domain and a CH3 domain and the antibody light chain comprises a CL domain, either kappa or lambda.

The antibody can be of any class (e.g. IgG, IgE, IgM, IgD and IgA) or subclass of immunoglobulin molecule. In one embodiment the antibody for use in the present invention is of IgG class and may be selected from any of the IgG subclasses IgG1, IgG2, IgG3 or IgG4. Differential ADCC induction by different IgG isotypes is dependent on the affinity of these residues to FcyRs. In humans, IgG1 and IgG3 are known to induce effector functions whereas IgG2 and IgG4 induce effector functions weakly.

In a preferred embodiment the antibody for use in the present invention is an IgG2 or IgG4 antibody which induces effector functions weakly, including ADCC. It is particularly preferred for the antibody for use in treating fibrotic disease in the present invention to have limited effector function because the use of an antibody with effector function may cause enhanced depletion of cells expressing CSF-1R including macrophages which has potential to add to side effects in the patient.

The antibody for use in the present invention may include one or more mutations to alter the activity of the antibody. Angal et al (Angal,S., King,D.J., Bodmer,M.W., Turner,A., Lawson,A.D., Roberts,G., Pedley,B., and Adair,J.R. 1993. A single amino acid substitution abolishes the heterogeneity of chimeric mouse/human (IgG4) antibody. Mol. Immunol. 30:105-108) describes a site directed mutagenesis approach to minimize half-molecule formation of IgG4 antibodies. In this report, a single amino acid substitution within the core hinge, S241P, resulted in substantially less half-molecule formation. Accordingly, in the embodiment where the antibody is an IgG4 antibody, the antibody may include the mutation S241P.

It will also be understood by one skilled in the art that antibodies may undergo a variety of posttranslational modifications. The type and extent of these modifications often depends on the host cell line used to express the antibody as well as the culture conditions. Such modifications may include variations in glycosylation, methionine oxidation, diketopiperazine formation, aspartate isomerization and asparagine deamidation. A frequent modification is the loss of a carboxy-terminal basic residue (such as lysine or arginine) due to the action of carboxypeptidases (as described in Harris, RJ. Journal of Chromatography 705:129-134, 1995). Accordingly, the C-terminal lysine of the antibody heavy chain may be absent.

This specific region or epitope of the human CSF-1R polypeptide can be identified by any suitable epitope mapping method known in the art in combination with any one of the antibodies provided by the present invention. Examples of such methods include screening peptides of varying lengths derived from CSF-1R for binding to the antibody of the present invention with the smallest fragment that can specifically bind to the antibody containing the sequence of the epitope recognised by the antibody. The CSF-1R peptides may be produced synthetically or by proteolytic digestion of the CSF-1R polypeptide. Peptides that bind the antibody can be identified by, for example, mass spectrometric analysis. In another example, NMR spectroscopy or X-ray crystallography can be used to identify the epitope bound by an antibody of the present invention. Once identified, the epitopic fragment which binds an antibody of the present invention can be used, if required, as an immunogen to obtain additional antibodies which bind the same epitope.

Biological molecules, such as antibodies or fragments, contain acidic and/or basic functional groups, thereby giving the molecule a net positive or negative charge. The amount of overall "observed" charge will depend on the absolute amino acid sequence of the entity, the local environment of the charged groups in the 3D structure and the environmental conditions of the molecule. The isoelectric point (pI) is the pH at which a particular molecule or solvent accessible surface thereof carries no net electrical charge. In one example, the CSF-1R antibody and fragments may be engineered to have an appropriate isoelectric point. This may lead to antibodies and/or fragments with more robust properties, in particular suitable solubility and/or stability profiles and/or improved purification characteristics.

Thus in one aspect the invention provides a humanised CSF-1R antibody engineered to have an isoelectric point different to that of the originally identified antibody. The antibody may, for example be engineered by replacing an amino acid residue such as replacing an acidic amino acid residue with one or more basic amino acid residues. Alternatively, basic amino acid residues may be introduced or acidic amino acid residues can be removed. Alternatively, if the molecule has an unacceptably high pI value acidic residues may be introduced to lower the pI, as required. It is important that when manipulating the pI care must be taken to retain the desirable activity of the antibody or fragment. Thus in one embodiment the engineered antibody or fragment has the same or substantially the same activity as the "unmodified" antibody or fragment.

Programs such as ** ExPASY http://www.expasy.ch/tools/pi tool.html, and
http://www.iut-arles.up.univ-mrs.fr/w3bb/d_abim/compo-p.html, may be used to predict the isoelectric point of the antibody or fragment.

It will be appreciated that the affinity of antibodies provided by the present invention may be altered using any suitable method known in the art. The present invention therefore also relates to variants of the antibody molecules of the present invention, which have an improved affinity for CSF-1R. Such variants can be obtained by a number of affinity maturation protocols including mutating the CDRs (Yang et al., J. Mol. Biol., 254, 392-403, 1995), chain shuffling (Marks et al., Bio/Technology, 10, 779-783, 1992), use of mutator strains of *E. coli* (Low et al., J. Mol. Biol., 250, 359-368, 1996), DNA shuffling (Patten et al., Curr. Opin. Biotechnol., 8, 724-733, 1997), phage display (Thompson et al., J. Mol. Biol., 256, 77-88, 1996) and sexual PCR (Crameri et al., Nature, 391, 288-291, 1998). Vaughan *et al.* (*supra*) discusses these methods of affinity maturation.

If desired an antibody for use in the present invention may be conjugated to one or more effector molecule(s). It will be appreciated that the effector molecule may comprise a single effector molecule or two or more such molecules so linked as to form a single moiety that can be attached to the antibodies of the present invention. Where it is desired to obtain an antibody fragment linked to an effector molecule, this may be prepared by standard chemical or recombinant DNA procedures in which the antibody fragment is linked either directly or via a coupling agent to the effector molecule. Techniques for conjugating such effector molecules to antibodies are well known in the art (see, Hellstrom et al., Controlled Drug Delivery, 2nd Ed., Robinson et al., eds., 1987, pp. 623-53; Thorpe et al., 1982 , Immunol. Rev., 62:119-58 and Dubowchik et al., 1999, Pharmacology and Therapeutics, 83, 67-123). Particular chemical procedures include, for example, those described in WO 93/06231, WO 92/22583, WO 89/00195, WO 89/01476 and WO 03/031581. Alternatively, where the effector molecule is a protein or polypeptide the linkage may be achieved using recombinant DNA procedures, for example as described in WO 86/01533 and EP0392745.

The term effector molecule as used herein includes, for example, antineoplastic agents, drugs, toxins, biologically active proteins, for example enzymes, other antibody or antibody fragments, synthetic or naturally occurring polymers, nucleic acids and fragments thereof e.g. DNA, RNA and fragments thereof, radionuclides, particularly radioiodide, radioisotopes, chelated metals, nanoparticles and reporter groups such as fluorescent compounds or compounds which may be detected by NMR or ESR spectroscopy.

Examples of effector molecules may include cytotoxins or cytotoxic agents including any agent that is detrimental to (*e.g.* kills) cells. Examples include combrestatins, dolastatins, epothilones, staurosporin, maytansinoids, spongistatins, rhizoxin, halichondrins, roridins, hemiasterlins, taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof.

Effector molecules also include, but are not limited to, antimetabolites (*e.g*. methotrexate, 6-mercaptopurine, 6-thioguanine, cytarabine, 5-fluorouracil decarbazine), alkylating agents (*e.g.* mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g*. daunorubicin (formerly daunomycin) and doxorubicin), antibiotics (*e.g*. dactinomycin (formerly actinomycin), bleomycin, mithramycin, anthramycin (AMC), calicheamicins or duocarmycins), and anti-mitotic agents (*e.g*. vincristine and vinblastine).

Other effector molecules may include chelated radionuclides such as ¹¹¹In and ⁹⁰Y, Lu¹⁷⁷, Bismuth²¹³, Californium²⁵², Iridium¹⁹² and Tungsten¹⁸⁸/Rhenium¹⁸⁸; or drugs such as but not limited to, alkylphosphocholines, topoisomerase I inhibitors, taxoids and suramin.

Other effector molecules include proteins, peptides and enzymes. Enzymes of interest include, but are not limited to, proteolytic enzymes, hydrolases, lyases, isomerases, transferases. Proteins, polypeptides and peptides of interest include, but are not limited to, immunoglobulins, toxins such as abrin, ricin A, pseudomonas exotoxin, or diphtheria toxin, a protein such as insulin, tumour necrosis factor, α-interferon, β-interferon, nerve growth factor, platelet derived growth factor or tissue plasminogen activator, a thrombotic agent or an anti-angiogenic agent, e.g. angiostatin or endostatin, or, a biological response modifier such as a lymphokine, interleukin-1 (IL-1), interleukin-2 (IL-2), granulocyte macrophage colony stimulating factor (GM-CSF), granulocyte colony stimulating factor (G-CSF), nerve growth factor (NGF) or other growth factor and immunoglobulins.

Other effector molecules may include detectable substances useful for example in diagnosis. Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include ¹²⁵I, ¹³¹I, ¹¹¹In and ⁹⁹Tc.

In another example the effector molecule may increase the half-life of the antibody *in vivo,* and/or reduce immunogenicity of the antibody and/or enhance the delivery of an antibody across an epithelial barrier to the immune system. Examples of suitable effector molecules of this type include polymers, albumin, albumin binding proteins or albumin binding compounds such as those described in WO05/117984.

In one embodiment a half-life provided by an effector molecule which is independent of CSF-1R is advantageous.

Where the effector molecule is a polymer it may, in general, be a synthetic or a naturally occurring polymer, for example an optionally substituted straight or branched chain polyalkylene, polyalkenylene or polyoxyalkylene polymer or a branched or unbranched polysaccharide, e.g. a homo- or hetero- polysaccharide.

Specific optional substituents which may be present on the above-mentioned synthetic polymers include one or more hydroxy, methyl or methoxy groups.

Specific examples of synthetic polymers include optionally substituted straight or branched chain poly(ethyleneglycol), poly(propyleneglycol) poly(vinylalcohol) or derivatives thereof, especially optionally substituted poly(ethyleneglycol) such as methoxypoly(ethyleneglycol) or derivatives thereof.

Specific naturally occurring polymers include lactose, amylose, dextran, glycogen or derivatives thereof.

In one embodiment the polymer is albumin or a fragment thereof, such as human serum albumin or a fragment thereof.

"Derivatives" as used herein is intended to include reactive derivatives, for example thiol-selective reactive groups such as maleimides and the like. The reactive group may be linked directly or through a linker segment to the polymer. It will be appreciated that the residue of such a group will in some instances form part of the product as the linking group between the antibody fragment and the polymer.

The size of the polymer may be varied as desired, but will generally be in an average molecular weight range from 500Da to 50000Da, for example from 5000 to 40000Da such as from 20000 to 40000Da. The polymer size may in particular be selected on the basis of the intended use of the product for example ability to localize to certain tissues such as tumors or extend circulating half-life (for review see Chapman, 2002, Advanced Drug Delivery Reviews, 54, 531-545). Thus, for example, where the product is intended to leave the circulation and penetrate tissue, for example for use in the treatment of a tumour, it may be advantageous to use a small molecular weight polymer, for example with a molecular weight of around 5000Da. For applications where the product remains in the circulation, it may be advantageous to use a higher molecular weight polymer, for example having a molecular weight in the range from 20000Da to 40000Da.

Suitable polymers include a polyalkylene polymer, such as a poly(ethyleneglycol) or, especially, a methoxypoly(ethyleneglycol) or a derivative thereof, and especially with a molecular weight in the range from about 15000Da to about 40000Da.

In one example antibodies for use in the present invention are attached to poly(ethyleneglycol) (PEG) moieties. In one particular example the antibody is an antibody fragment and the PEG molecules may be attached through any available amino acid side-chain or terminal amino acid functional group located in the antibody fragment, for example any free amino, imino, thiol, hydroxyl or carboxyl group. Such amino acids may occur naturally in the antibody fragment or may be engineered into the fragment using recombinant DNA methods (see for example US 5,219,996; US 5,667,425; WO98/25971, WO2008/038024). In one example the antibody molecule of the present invention is a modified Fab fragment wherein the modification is the addition to the C-terminal end of its heavy chain one or more amino acids to allow the attachment of an effector molecule. Suitably, the additional amino acids form a modified hinge region containing one or more cysteine residues to which the effector molecule may be attached. Multiple sites can be used to attach two or more PEG molecules.

Suitably PEG molecules are covalently linked through a thiol group of at least one cysteine residue located in the antibody fragment. Each polymer molecule attached to the modified antibody fragment may be covalently linked to the sulphur atom of a cysteine residue located in the fragment. The covalent linkage will generally be a disulphide bond or, in particular, a sulphur-carbon bond. Where a thiol group is used as the point of attachment appropriately activated effector molecules, for example thiol selective derivatives such as maleimides and cysteine derivatives may be used. An activated polymer may be used as the starting material in the preparation of polymer-modified antibody fragments as described above. The activated polymer may be any polymer containing a thiol reactive group such as an α-halocarboxylic acid or ester, e.g. iodoacetamide, an imide, e.g. maleimide, a vinyl sulphone or a disulphide. Such starting materials may be obtained commercially (for example from Nektar, formerly Shearwater Polymers Inc., Huntsville, AL, USA) or may be prepared from commercially available starting materials using conventional chemical procedures. Particular PEG molecules include 20K methoxy-PEG-amine (obtainable from Nektar, formerly Shearwater; Rapp Polymere; and SunBio) and M-PEG-SPA (obtainable from Nektar, formerly Shearwater).

In one embodiment, the antibody is a modified Fab fragment, Fab' fragment or diFab which is PEGylated, *i.e.* has PEG (poly(ethyleneglycol)) covalently attached thereto, *e.g.* according to the method disclosed in EP 0948544 or EP1090037 [see also "Poly(ethyleneglycol) Chemistry, Biotechnical and Biomedical Applications", 1992, J. Milton Harris (ed), Plenum Press, New York, "Poly(ethyleneglycol) Chemistry and Biological Applications", 1997, J. Milton Harris and S. Zalipsky (eds), American Chemical Society, Washington DC and "Bioconjugation Protein Coupling Techniques for the Biomedical Sciences", 1998, M. Aslam and A. Dent, Grove Publishers, New York; Chapman, A. 2002, Advanced Drug Delivery Reviews 2002, 54:531-545]. In one example PEG is attached to a cysteine in the hinge region. In one example, a PEG modified Fab fragment has a maleimide group covalently linked to a single thiol group in a modified hinge region. A lysine residue may be covalently linked to the maleimide group and to each of the amine groups on the lysine residue may be attached a methoxypoly(ethyleneglycol) polymer having a molecular weight of approximately 20,000Da. The total molecular weight of the PEG attached to the Fab fragment may therefore be approximately 40,000Da.

Particular PEG molecules include 2-[3-(N-maleimido)propionamido]ethyl amide of N,N'-bis(methoxypoly(ethylene glycol) MW 20,000) modified lysine, also known as PEG2MAL40K (obtainable from Nektar, formerly Shearwater).

Alternative sources of PEG linkers include NOF who supply GL2-400MA3 (wherein m in the structure below is 5) and GL2-400MA (where m is 2) and n is approximately 450:

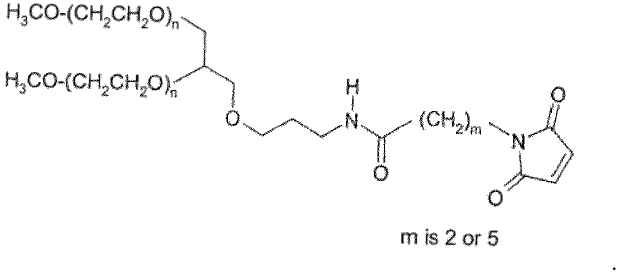

That is to say each PEG is about 20,000Da.

Thus in one embodiment the PEG is 2,3-Bis(methylpolyoxyethylene-oxy)-1-{[3-(6-maleimido-1-oxohexyl)amino]propyloxy}hexane (the 2 arm branched PEG, -CH₂) ₃NHCO(CH₂)₅-MAL, Mw 40,000 known as SUNBRIGHT GL2-400MA3.

Further alternative PEG effector molecules of the following type:

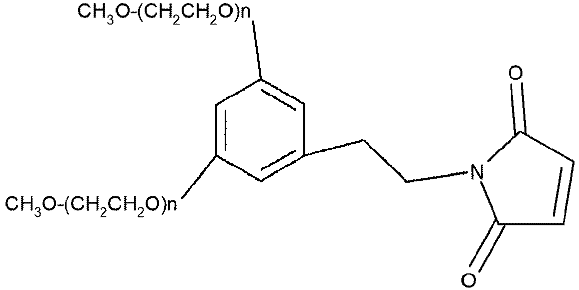

In one embodiment there is provided an antibody, such as a full length antibody, which is PEGylated (for example with a PEG described herein), attached through a cysteine amino acid residue at or about amino acid 226 in the chain, for example amino acid 226 of the heavy chain (by sequential numbering).

In one embodiment the present disclosure provides a Fab'PEG molecule comprising one or more PEG polymers, for example 1 or 2 polymers such as a 40kDa polymer or polymers.

Fab-PEG molecules according to the present disclosure may be particularly advantageous in that they have a half-life independent of the Fc fragment.

In one embodiment there is provided a scFv conjugated to a polymer, such as a PEG molecule, a starch molecule or an albumin molecule.

In one embodiment the antibody or fragment is conjugated to a starch molecule, for example to increase the half-life. Methods of conjugating start to a protein as described in US 8,017,739.

To identify antibodies inhibiting CSF-1R activity a number of different approaches may be taken by those skilled in the art. In one example inhibitors are identified by first identifying agents that interact with CSF-1R and subsequently testing those agents to identify those that inhibit CSF-1R activity. Agents that interact with CSF1-R may be identified using any suitable method, for example by using a cell-free or cell-based assay system where the CSF-1R polypeptide is contacted with a candidate agent and the ability of the candidate agent to interact with the polypeptide is determined. Preferably, the ability of a candidate agent to interact with a CSF-1R polypeptide is compared to a reference range or control. If desired, this assay may be used to screen a plurality (e.g. a library) of candidate agents using a plurality of CSF-1R polypeptide samples. In one example of a cell free assay, a first and second sample comprising native or recombinant CSF-1R polypeptide are contacted with a candidate agent or a control agent and the ability of the candidate agent to interact with the polypeptide is determined by comparing the difference in interaction between the candidate agent and control agent. Preferably, the polypeptide is first immobilized, by, for example, contacting the polypeptide with an immobilized antibody which specifically recognizes and binds it, or by contacting a purified preparation of polypeptide with a surface designed to bind proteins. The polypeptide may be partially or completely purified (e.g. partially or completely free of other polypeptides) or part of a cell lysate. Further, the polypeptide may be a fusion protein comprising the CSF1-R polypeptide or a biologically active portion thereof and a domain such as glutathionine-S-transferase or the Fc region of IgG1. Alternatively, the polypeptide can be biotinylated using techniques well known to those of skill in the art (e.g. biotinylation kit, Pierce Chemicals; Rockford, IL). The ability of the candidate agent to interact with the polypeptide can be determined by methods known to those of skill in the art for example, ELIS A, BIAcore™, Flow cytometry or fluorescent microvolume assay technology (FMAT). In another example where a cell-based assay is used, a population of cells expressing CSF-1 R is contacted with a candidate agent and the ability of the candidate agent to interact with the polypeptide is determined. Preferably, the ability of a candidate agent to interact with CSF-1 R is compared to a reference range or control. The cell, for example, can be of eukaryotic origin (e.g. yeast or mammalian) and can express the CSF-1 R polypeptide endogenously or be genetically engineered to express the polypeptide. In some instances, the CSF-1R polypeptide or the candidate agent is labelled, for example with a radioactive label (such as P, S or I) or a fluorescent label (such as fluorescein isothiocyanate, rhodamine, phycoerythrin, phycocyanin, allophycocyanin, o-phthaldehyde or fluorescamine) to enable detection of an interaction between a polypeptide and a candidate agent. Alternative methods such as ELISA, flow cytometry and FMAT may also be used. Agents which inhibit CSF-1R activity may be identified by any suitable method, for example by: (i) comparing the activity of CSF- 1R in the presence of a candidate agent with the activity of said polypeptide in the absence of the candidate agent or in the presence of a control agent; and (ii) determining whether the candidate agent inhibits activity of CSF-1R. Such assays can be used to screen candidate agents, in clinical monitoring or in drug development. As described above, agents may be pre-screened where appropriate (e.g. an antibody) to identify agents that interact with CSF-1R prior to screening those agents which bind for their ability to inhibit CSF-1R activity. In one example a cell-based assay system is used to identify agents capable of inhibiting the activity of CSF-1R.

In one particular example the assay used to identify inhibitors of CSF-1R activity is the standard *in vitro* colony stimulating assay of Metcalf, 1970, J. Cell.Physiol.76-89 in which CSF-1 is capable of stimulating the formation of macrophage colonies. Potential inhibitors are added to the assay and proliferation of macrophages is measured by any suitable method such as ³H thymidine incorporation or formazan dye conversion. Inhibition is therefore measured as a reduction in proliferation compared to controls.

Agents that inhibit the activity of CSF-1R may be identified or further tested, for example to determine therapeutically effective amounts in one or more animal models. Examples of suitable animals include, but are not limited to, mice, rats, rabbits, monkeys, guinea pigs, dogs and cats.

In another example, the inhibition of CSF-1R activity can be determined by monitoring an amelioration or improvement in disease symptoms, and/or a delayed onset or slowed progression of the disease. For example but without limitation, in the case of IPF this could be manifest as a reduction in the decline of lung function as measured by forced vital capacity, a reduction in the decline of diffusing capacity as measured by DLCO, or a reduction in the decline of the six minute walking distance. Inhibition of CSF-1R activity in patients with IPF can also be determined by monitoring clinical events such as exacerbations, defined as a rapid deterioration in disease activity in the absence of other causes. In the case of hepatic fibrosis, the enhanced liver fibrosis (ELF) test can be used to monitor disease by quantitatively measuring the amount of hyaluronic acid, amino-terminal pro-peptide of type III pro-collagen (PIIINP) and tissue inhibitor of metalloproteinase 1 (TIMP1) in serum and combining them through use of an algorithm to produce a single ELF score, the magnitude of which indicates disease severity. With regard to systemic sclerosis, the effects of CSF-1R inhibition could be manifest as reduced progression of the Modified Rodnan Skin Score and/or the Medsger Severity Scale, or at the level of individual organs, for example retardation of scleroderma associated interstitial lung disease. The effect of CSF-1R inhibition on the course of renal fibrosis, including but not limited to chronic kidney disease and post-transplant graft fibrosis, could be manifest as a reduction in the decline or improvement of glomerular filtration rate (GFR) and/or proteinuria.

Thus in one embodiment employing an inhibitor of CSF-1R may provide a reduction in one or more of the follow in the decline of lung function as measured by forced vital capacity or a reduction in the decline of distance covered in the six minute walk test in IPF patients. The enhanced liver fibrosis (ELF) test can be used to monitor liver fibrosis by quantitatively measuring the amount of hyaluronic acid, amino-terminal pro-peptide of type III pro-collagen (PIIINP) and tissue inhibitor of metalloproteinase 1 (TIMP1) in serum and combining them through use of an algorithm to produce a single ELF score, the magnitude of which indicates disease severity.

Techniques known to physicians familiar with fibrotic disease can be used to determine whether a candidate agent has altered one or more symptoms associated with the disease. A number of different models of fibrotic disease are known in the art. These include bleomycin-induced lung fibrosis, reviewed in Moore BB and Hogaboam CM, 2008, Am J Physiol Lung Cell Mol Physiol, 294, L152-60; Scotton CJ and Chambers RC, 2010 Am J Physiol Lung Cell Mol Physiol, 299, L439-41, adenosine deaminase deficient mouse model of adenosine-induced fibrosis (Chunn JL et al, 2005, J Immunol 175: 1937-46; Sun C_X et al, 2006, J Clin Invest, 116, 2173-82), radiation induced lung fibrosis, (Sharplin J and Franko AJ, 1989, Radiation Research 119, 1-14; Sharplin J and Franko AJ, 1989, Radiation Research 119, 15-31; Johnston CJ et al, 1998, Exp Lung Res, 24, 321-37; Haston CK and Travis EL, 1997, Cancer Res 57, 5286-91; Katoh H et al, 2010, J Radiat Res, 51, 325-332,), FITC induced lung fibrosis (Roberts SN et al, 1995, J Pathol, 176, 309-18; Christensen, P et al, 1999, Am J Pathol, 155, 1773-79), silica induced fibrosis in the mouse (Davis GS, et al, 1998, J Environ Pathol Toxicol Oncol 17, 81-97; Lakatos HF et al, 2006, Exp Lung Res 32, 181-99), the carbon tetrachloride (CCl4) model of liver fibrosis (Iredale JP et al, 1998, J Clin Invest, 102, 538-49), the bile duct ligation model of hepatic fibrosis (Stahelin BJ et al, 1999, Virchows Arch, 434, 333-39; Issa R et al, 2001, Gut, 48, 548-57), the UUO (unilateral ureteric obstruction) of kidney fibrosis (Klahr S and Pukerson ML, 1994, Am J Kidney Dis, 23, 219-23; Satoh M et al, 2001, J Am Soc Nephrol, 12, 317-25), the adriamycin induced model of chronic kidney disease (Tamaki, K et al, 1994, Kidney Int, 45, 525-36; Wang Y et al, 2000, Kidney Int, 58, 1797-1804 and reviewed by Lee VW and Harris DC, 2011, Nephrology, 16, 30-38), the 5/6 reduction model of chronic kidney disease (Griffin A et al, 1994, J Am Soc Nephrol, 4, 2023-31; the hypochlorous acid induced model of systemic sclerosis (Servettaz, A et al, 2009, J Immunol, 182(9), 5855-64), and alternative models of systemic sclerosis such as bleomycin induced skin fibrosis, tight skin mouse model, in which mice are heterozygous for a mutation in the fibrillin-1 gene and the sclerodermatous graft versus host disease model where the mice develop skin and pulmonary fibrosis, reviewed in Yamamoto, T, 2010, J Dermatol, 37, 26-41. Generally the different animal models exhibit excessive production and deposition of extracellular matrix including collagen in the affected organ or tissue which is the hallmark of fibrosis. Other measurements of fibrotic disease will vary between models as different organs are affected in the specific models. The elevation of protein and creatinine levels in urea may be measured in models of kidney fibrosis whilst in models of pulmonary fibrosis a histopathological score of the extent and severity of fibrosis, for example, the modified Ashcroft score, is often used (Hubner RH et al, 2008, Biotechniques 44: 507-17).

Monitoring techniques for fibrosis include lung function tests for pulmonary fibrosis, the ELF test for liver disease/fibrosis, the FibroTest (blood analysis of fibrosis markers) for liver disease and transient elastography (Fibroscan®) which is an imaging technology that provides a measure of liver stiffness, useful in detecting and assessing liver fibrosis. Analysis of tissue samples from a biopsy can also be performed.

As discussed herein, inhibitors of CSF-1R activity can be used in the treatment and/or prophylaxis of fibrotic disease. For such use the agents will generally be administered in the form of a pharmaceutical composition. Also provided is a pharmaceutical composition comprising an inhibitor of CSF-1R activity and a pharmaceutically acceptable diluent, excipient and /or carrier. The term 'treatment' includes either therapeutic or prophylactic therapy. When a reference is made herein to a method of treating or preventing a disease or condition using a particular inhibitor or combination of inhibitors, it is to be understood that such a reference is intended to include the use of that inhibitor or combination of inhibitors for the manufacture of a medicament for the treatment and/or prophylaxis of fibrotic disease. The composition will usually be supplied as part of a sterile, pharmaceutical composition that will normally include a pharmaceutically acceptable carrier. This composition may be in any suitable form (depending upon the desired method of administering it to a patient). The inhibitors of use in the invention are preferably administered to a subject orally or intrarectally but may also be administered by a variety of other routes such as transdermally, subcutaneously, intranasally, intravenously and intramuscularly. The most suitable route for administration in any given case will depend on the particular inhibitor, the subject, and the nature and severity of the disease and the physical condition of the subject.

The inhibitors of use may be administered in combination, e.g. simultaneously, sequentially or separately, with one or more other therapeutically active compounds, which may be for example an anti-fibrotic therapy such as Perfenidone or anticancer therapies.

Pharmaceutical compositions maybe conveniently presented in unit dose forms containing a predetermined amount of an active agent of the invention per dose. Such a unit may contain for example but without limitation, 1000mg/kg to 0.01mg/kg for example 750mg/kg to 0.1mg/kg, such as 100mg/kg to 1mg/kg depending on the condition being treated, the route of administration and the age, weight and condition of the subject.

Pharmaceutically acceptable carriers for use in the invention may take a wide variety of forms depending, e.g. on the route of administration.

Compositions for oral administration may be liquid or solid. Oral liquid preparations may be in the form of, for example, aqueous or oily suspensions, solutions, emulsions, syrups or elixirs, or may be presented as a dry product for reconstitution with water or other suitable vehicle before use. Oral liquid preparations may contain suspending agents as known in the art. In the case of oral solid preparations such as powders, capsules and tablets, carriers such as starches, sugars, microcrystalline cellulose, granulating agents, lubricants, binders, disintegrating agents, and the like may be included. Because of their ease of administration, tablets and capsules represent the most advantageous oral dosage unit form in which case solid pharmaceutical carriers are generally employed.

In addition to the common dosage forms set out above, active agents of the invention may also be administered by controlled release means and/or delivery devices. Tablets and capsules may comprise conventional carriers or excipients such as binding agents for example, syrup, acacia, gelatin, sorbitol, tragacanth, or polyvinylpyrrolidone; fillers, for example lactose, sugar, maize-starch, calcium phosphate, sorbitol or glycine; tableting lubricants, for example magnesium stearate, talc, polyethylene glycol or silica; disintegrants, for example potato starch; or acceptable wetting agents such as sodium lauryl sulphate. The tablets may be coated by standard aqueous or non-aqueous techniques according to methods well known in normal pharmaceutical practice. Pharmaceutical compositions of the present invention suitable for oral administration may be presented as discrete units such as capsules, cachets or tablets, each containing a predetermined amount of the active agent, as a powder or granules, or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active agent with the carrier, which constitutes one or more necessary ingredients. In general, the compositions are prepared by uniformly and intimately admixing the active agent with liquid carriers or finely divided solid carriers or both, and then, if necessary, shaping the product into the desired presentation. For example, a tablet may be prepared by compression or moulding, optionally with one or more accessory ingredients.

Pharmaceutical compositions suitable for parenteral administration may be prepared as solutions or suspensions of the active agents of the invention in water suitably mixed with a surfactant such as hydroxypropylcellulose. Dispersions can also be prepared in glycerol, liquid polyethylene glycols, and mixtures thereof in oils. Under ordinary conditions of storage and use, these preparations contain a preservative to prevent the growth of microorganisms. The pharmaceutical forms suitable for injectable use include aqueous or non-aqueous sterile injection solutions which may contain anti-oxidants, buffers, bacteriostats and solutes which render the composition isotonic with the blood of the intended recipient, and aqueous and non-aqueous sterile suspensions which may include suspending agents and thickening agents. Extemporaneous injection solutions, dispersions and suspensions may be prepared from sterile powders, granules and tablets. Pharmaceutical compositions can be administered with medical devices known in the art. For example, in a preferred embodiment, a pharmaceutical composition of the invention can be administered with a needleless hypodermic injection device, such as the devices disclosed in US 5,399,163; 5,383,851; 5,312,335; 5,064,413; 4,941,880; 4,790,824; or 4,596,556. Examples of well-known implants and modules useful in the present invention include: US 4,487,603, which discloses an implantable micro-infusion pump for dispensing medication at a controlled rate; US 4,486,194, which discloses a therapeutic device for administering medicaments through the skin; US 4,447,233, which discloses a medication infusion pump for delivering medication at a precise infusion rate; US 4,447,224, which discloses a variable flow implantable infusion apparatus for continuous drug delivery; US 4,439,196, which discloses an osmotic drug delivery system having multi-chamber compartments; and US 4,475,196, which discloses an osmotic drug delivery system. Many other such implants, delivery systems, and modules are known to those skilled in the art.

Pharmaceutical compositions adapted for topical administration may be formulated as ointments, creams, suspensions, lotions, powders, solutions, pastes, gels, impregnated dressings, sprays, aerosols or oils, transdermal devices, dusting powders, and the like. These compositions may be prepared via conventional methods containing the active agent. Thus, they may also comprise compatible conventional carriers and additives, such as preservatives, solvents to assist drug penetration, emollients in creams or ointments and ethanol or oleyl alcohol for lotions. Such carriers may be present as from about 1 percent up to about 98 percent of the composition. More usually they will form up to about 80 percent of the composition. As an illustration only, a cream or ointment is prepared by mixing sufficient quantities of hydrophilic material and water, containing from about 5-10 percent by weight of the compound, in sufficient quantities to produce a cream or ointment having the desired consistency. Pharmaceutical compositions adapted for transdermal administration may be presented as discrete patches intended to remain in intimate contact with the epidermis of the recipient for a prolonged period of time. For example, the active agent may be delivered from the patch by iontophoresis. For applications to external tissues, for example the mouth and skin, the compositions are preferably applied as a topical ointment or cream. When formulated in an ointment, the active agent may be employed with either a paraffinic or a water-miscible ointment base. Alternatively, the active agent may be formulated in a cream with an oil-in-water cream base or a water-in-oil base. Pharmaceutical compositions adapted for topical administration in the mouth include lozenges, pastilles and mouth washes. Pharmaceutical compositions adapted for topical administration to the eye include eye drops wherein the active agent is dissolved or suspended in a suitable carrier, especially an aqueous solvent. They also include topical ointments or creams as above. Pharmaceutical compositions suitable for rectal administration wherein the carrier is a solid are most preferably presented as unit dose suppositories. Suitable carriers include cocoa butter or other glyceride or materials commonly used in the art, and the suppositories may be conveniently formed by admixture of the combination with the softened or melted carrier(s) followed by chilling and shaping moulds. They may also be administered as enemas.

The dosage to be administered of an inhibitor of CSF-1R activity will vary according to the particular inhibitor, the type of fibrotic disease, the subject, and the nature and severity of the disease and the physical condition of the subject, and the selected route of administration; the appropriate dosage can be readily determined by a person skilled in the art. For the treatment and/or prophylaxis of fibrotic disease in humans and animals pharmaceutical compositions comprising antibodies can be administered to patients (e.g., human subjects) at therapeutically or prophylactically effective dosages (e.g. dosages which result in inhibition of fibrotic disease and/or relief of fibrotic disease symptoms) using any suitable route of administration, such as injection and other routes of administration known in the art for clinical products, such as antibody-based clinical products. The compositions may contain at least 0.05 percent by weight, for example 0.5 to 50 percent by weight such as 1 to 10 percent by weight, or more, by weight, of the inhibitor of the invention, depending on the method of administration. It will be recognized by one of skill in the art that the optimal quantity and spacing of individual dosages of an inhibitor of the invention will be determined by the nature and extent of the condition being treated, the form, route and site of administration, and the age and condition of the particular subject being treated, and that a physician will ultimately determine appropriate dosages to be used. This dosage may be repeated as often as appropriate. If side effects develop the amount and/or frequency of the dosage can be altered or reduced, in accordance with normal clinical practice.

The invention will now be described with reference to the following examples, which are merely illustrative and should not in any way be construed as limiting the scope of the present invention.

### FIGURES

- **Figure 1A**: shows the amino acid sequence for human CSF-1R
- **Figure 1B**: shows the amino acid sequence for certain variants of CSF-1R
- **Figure 1C**: shows an amino acid sequence of CSF-1R suitable for expression on cell surfaces
- **Figure 2**: shows flow cytometry data confirming that receptor-bound CSF-1 on M-NFS-60 cells was detectable by this method
- **Figure 3**: shows Ab535, either immobilised on the surface of the tissue culture well, or free in solution, did not support the proliferation of M-NFS-60 cells in the absence of CSF-1 at the concentrations used
- **Figure 4a**: shows that treatment of bleomycin-induced lung fibrosis with Ab535 reduced the amount of BAL fluid collagen compared to treatment with the isotype control.
- **Figure 4b**: shows that treatment of bleomycin-induced lung fibrosis with Ab535 reduced the Ashcroft score of the samples compared to treatment with the isotype control demonstrating that the mice treated with Ab535 had improved fibrotic pathology.
- **Figure 4c**: shows that treatment of bleomycin-induced lung fibrosis with Ab535 reduced the amount of albumin in BAL fluid compared to treatment with the isotype control which thus shows that the integrity of the pulmonary barrier of the mice treated with Ab535 was improved.
- **Figure 4d**: shows that treatment of bleomycin-induced lung fibrosis with Ab535 reduced the number of macrophages in the BAL fluid.
- **Figure 5**: shows representative images of the histopathological analysis of lungs from saline control, bleomycin plus isotype control and bleomycin plus Ab 535 treated animals. Animals treated with Ab 535 had a greatly reduced fibrosis of the lungs compared to the isotype control, bleomycin treated animals.
- **Figure 6a**: shows that treatment of ADA-deficient mice with induced lung fibrosis, with Ab535, reduced BALF collagen concentration compared to treatment with the isotype control.
- **Figure 6b**: shows that treatment of ADA-deficient mice with induced lung fibrosis, with Ab535, reduced the Ashcroft score of the samples compared to treatment with the isotype control demonstrating that the mice treated with Ab535 had improved fibrotic pathology.
- **Figure 6c**: shows that treatment of ADA-deficient mice with induced lung fibrosis, with Ab535, reduced the amount of albumin in the BAL compared to treatment with the isotype control demonstrating that the integrity of the pulmonary barrier of the mice treated with Ab535 was improved.
- **Figure 6d**: shows that treatment of ADA-deficient mice with induced lung fibrosis, with Ab535, reduced the number of macrophages in the BAL fluid.
- **Figure 7**: shows representative images of the histopathological analysis of lung tissue from ADA knockout mice and wild type mice either treated with Ab535 or an isotype control antibody (mouse IgG1). Lung tissue sections were stained with Masson's trichrome. Treatment of ADA knockout mice with Ab535 greatly reduced the amount of fibrotic tissue and collagen deposition.

### Example 1 Isolation of an anti-mouse CSF-1R antibody

2 rabbits received 5 immunisations with cells transiently expressing residues 1-512 of mouse CSF-1R **(Uniprot Entry p09581).** Antibody response was monitored in an ELISA using Nunc Maxisorp plates coated with 2 µg/ml mouse CSF-1R-Rabbit Fc. Sera titres out to 1:100,000 dilution were observed with both rabbits. Binding of sera to M-NFS-60 cells (Metcalf et al., 1970) was also determined by FACS. Median FL1 was plotted against antibody dilution. Binding out to a dilution of 1:10,000 was observed.

Using the CSF-1-dependent M-NFS-60 cell line (Metcalf et al., 1970), it was shown that sera from both rabbits could block CSF-1-dependent cell survival out to a dilution of 1:100 (data not shown).

One hundred 96-well plates were seeded with 1000-5000 rabbit PBMCs per well and grown for 1 week at 37C in the presence of EL4-B5 mouse thymoma cells and rabbit T cell conditioned media (TSN). Antibody-containing supernatants were then screened in an FMAT assay using M-NFS-60 cells and anti-rabbit Fc-specific Cy5 conjugate. 678 CSF-1R-binders were identified. From these, approximately 3% blocked CSF-1-dependent M-NFS-60 proliferation in a cell assay. Binders were also screened in an ELISA using plates coated with 2 µg/ml CSF-1R-rabbit Fc and binding revealed with an anti-rabbit F(ab')2-HRP conjugate. Binders were also screened in an ELISA-based solid-phase blocking assay. For this assay, Nunc Maxisorp 384-well plates were coated with 0.5 µg/ml CSF-1R-rabbit Fc and subsequently blocked in PBS/0.1% Tween-20/1% PEG20000. Plates were washed before culture supernatant was then added to the plates and incubated for >1hour at room temperature. CSF-1 was then added into the supernatant at a final concentration of 10 ng/ml and incubated for a further 1 hour at room temperature. Plates were washed and then incubated with 0.5 µg/ml biotinylated goat anti-mouse CSF-1 antibody (R&D systems). CSF-1 binding to receptor was revealed using streptavidin-HRP. Eight wells were selected for progression. All demonstrated binding to cells and protein and blocked in the M-NFS-60 assay and in the ELISA.

A fluorescence-based method was used to identify the antigen-specific B cells from a positive well. Immunoglobulin-secreting B cells from a positive well were mixed with streptavidin beads coated with biotinylated mouse CSF-1R-rabbit Fc and a goat F(ab')2 anti-rabbit F(ab')2 fragment-specific FITC conjugate (Jackson ImmunoResearch). After incubation at 37C for 1 hour, antibody secreted from antigen-specific B cells was captured on beads in the vicinity of that B cell. The presence of the FITC conjugate resulted in the labelling of antibody-coated beads and the formation of fluorescent foci around the antigen-specific B cells. These individual B cells, identified using a fluorescent microscope, were then picked with a micromanipulator and deposited into a PCR tube. Antibody variable region genes were recovered from single cells by reverse transcription (RT)-PCR. Rabbit V-regions were then expressed as human IgG4 chimeric antibodies or Fabs in a CHO transient expression system. 4 out of 8 wells produced recombinant anti-CSF-1R antibodies. Neutralisation activity with the recombinant antibodies was then confirmed in the M-NFS-60 assay. BIAcore was also performed using the Fab molecules to determine affinity for CSF-1R rabbit Fc (Table 1). Based on neutralisation activity and affinity, Ab535 was selected as the anti-mouse CSF-1R reagent. This antibody was subsequently murinised, expressed in a mammalian system as full length murine IgG1 and purified.

### BIAcore Method

Antibodies were tested for their ability to bind CSF-1R in a BIAcore assay by measurement of binding kinetics to a purified recombinant CSF-1R/Fc fusion protein.

The assay format was capture of the anti-CSF-1R antibodies by immobilised anti-human IgG,F(ab')₂, then a titration of hCSF-1R/Fc over the captured surface. BIA (Biamolecular Interaction Analysis) was performed using a BIAcore 3000 (GE Healthcare Bio-Sciences AB). All experiments were performed at 25 °C Affinipure F(ab')₂ fragment goat anti-human IgG, F(ab')₂ fragment specific (Jackson ImmunoResearch) was immobilised on a CM5 Sensor Chip (GE Healthcare Bio-Sciences AB) via amine coupling chemistry to a level of ∼6000 response units (RU). HBS-EP buffer (10 mM HEPES pH 7.4, 0.15 M NaCl, 3 mM EDTA, 0.005% Surfactant P20, GE Healthcare Bio-Sciences AB) was used as the running buffer with a flow rate of 10 µl/min. An injection of an anti-CSF-1R antibody was performed to give a capture level of approximately 100 RU on the immobilised anti-human IgG,F(ab)₂.

Recombinant human CSF-1R/Fc was titrated (R&D Systems), by doubling dilution, from 2.5 nM to 78 pM over the captured anti-CSF-lR antibody at a flow rate of 30 µl/min for 3 min followed by a 8 min dissociation phase. These sensorgrams were used to generate the association rate. The surface was regenerated at a flow rate of 10 µl/min by two sequential 10 µl injections of 40 mM HCl followed by a 5 µl injection of 10 mM NaOH. Double referenced background subtracted binding curves were analysed using the BIAevaluation software (version 4.1) following standard procedures. Kinetic parameters were determined from the fitting algorithm.

The results for Ab553 are shown in **Table 1.**

**Table 1**

| **ANTIBODY REF:** | **On Rate ka (M⁻¹s⁻¹)** | **Off Rate kd (s⁻¹)** | **Affinity Constant K_{D}** |
|---|---|---|---|
| Ab 535 | 8.05 ± 0.01 e 6 | 1.21 + 0.15 e -5 | 1.50 pM |

### Example 2 In vitro analysis of Ab535

### Ab535 BLOCKS BINDING OF MURINE CSF-1 TO CSF-1 RECEPTOR-POSITIVE CELL LINES IN VITRO

The capacity of receptor-bound Ab535 to prevent CSF-1 from binding to CSF-1R was investigated. A murine CSF-1R-positive cell line, M-NFS-60, was used in assay in which cells were pre-incubated with Ab535 or a control antibody prior to exposure to CSF-1. Receptor-bound CSF-1 was then detected using a fluorescently-labelled antibody and flow cytometry. An Ab535-dependent decrease in cell fluorescence intensity was interpreted as indicating a capacity of receptor-bound Ab535 to prevent the binding of CSF-1 to CSF-1R.

M-NFS-60 cells (LGC Promochem, Teddington, UK) were maintained in suspension in basal RPMI medium (Invitrogen) supplemented with 10% fetal calf serum (PAA), Hepes (Invitrogen, 10 mM final concentration), Sodium Pyruvate (Invitrogen, 1 mM final concentration) Glucose (Sigma-Aldrich, 4.5 g/l final concentration), Sodium bicarbonate (Sigma-Aldrich, 1.5% final concentration), beta-mercaptoethanol (Sigma-Aldrich, 0.05 mM final concentration), recombinant murine CSF-1 (Preprotech, 3.3 ng/ml final concentration).

M-NFS-60 cells were prepared in flow buffer (PBS (Invitrogen) supplemented with 0.2% BSA (Sigma-Aldrich), 0.09% sodium azide (Sigma-Aldrich)) at a concentration of 1x10E6 cells/ml. Serial dilutions of Ab535 or a murine IgG1 isotype control antibody were prepared in flow buffer and added to 100µl aliquots of cells to achieve final concentrations of 20, 5, 1.25, 0.32, 0.08 µg/ml in 200 µl volume and were incubated on ice for 1 hour. Thereafter, cells were washed twice in flow buffer, incubated with 0.5 µg/ml recombinant murine CSF-1 for 30 mins on ice and washed twice in flow buffer. To detect receptor-bound CSF-1, cells were incubated with a biotinylated anti-murine CSF-1 antibody (R&D Systems, 5µg/ml) for 30 minutes on ice, washed twice in flow buffer and bound antibody labelled by incubation with Alexafluor 488-conjugated straptavidin (Invitrogen, 1:200 dilution) on ice for 15 minutes prior to a final washing step and resuspension of cells in 500 µl flow buffer. Cells were assessed for fluorescence by flow cytometry using a FACSCaliber flow cytometer (Becton Dickinson) and data were analysed using WinMDI software.

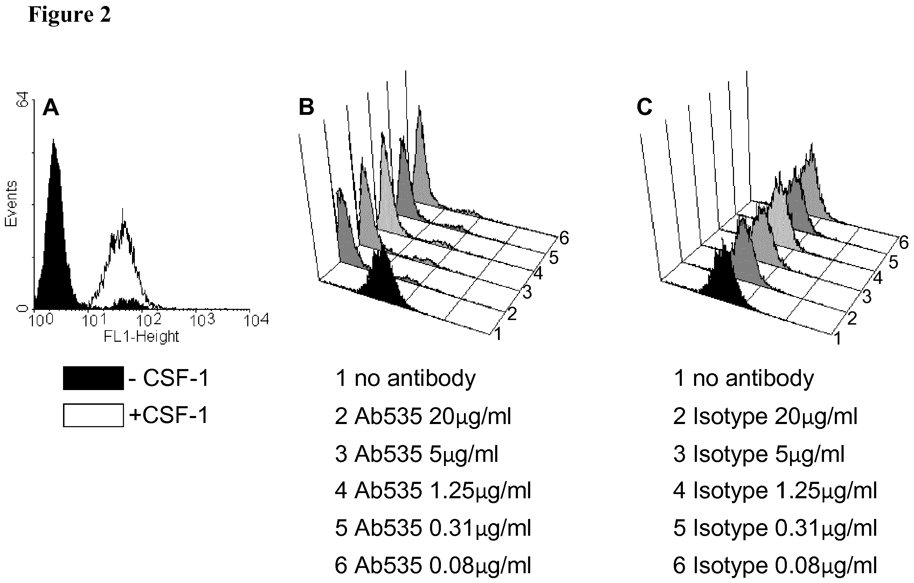

Analysis of flow cytometry data confirmed that receptor-bound CSF-1 on M-NFS-60 cells was detectable by this method **(****Figure 2a****).** Incubation of cells with Ab535 at any of the concentrations used, prior to addition of CSF-1 prevented binding of CSF-1 to CSF-1R as **(****Figure 2b****).** Incubation of cells with an isotype control antibody at any of the concentrations used had no effect on CSF-1 binding and detection **(****Figure 2c****).**

### BINDING OF Ab535 TO CSF-1R DOES NOT SUBSTITUTE FOR CSF-1 IN THE PROLIFERATION AND SURVIVAL OF A CSF-1 DEPENDENT CELL LINE IN VITRO

The capacity of Ab535 to substitute for CSF-1 in supporting the survival and proliferation of CSF-1-dependent cells was investigated using M-NFS-60 cells in a cell proliferation assay that included exposing cells to Ab535 free in solution and immobilised on plastic.

To assess the capacity of immobilised Ab535 to support CSF-1-dependent cells, wells of a 96-well round-bottomed tissue culture plate were pre-coated with Ab535 or an irrelevant isotype control antibody for 24 hours prior to initiating the cell proliferation assay by addition of 50 µl per well of a solution containing 10 µg/ml or 1 µg/ml of antibody in PBS and incubation at 4°C. Wells were aspirated and unbound antibody removed by washing each well twice with 100 µl PBS.

Proliferating M-NFS-60 cells, maintained as described above, were seeded into 96-well round-bottomed tissue culture plates at a density of 10,000 cells per well in 50 µl of growth medium without CSF-1. A further 50 µl growth medium, supplemented with either recombinant murine CSF-1, Ab535 or with no supplements, was added to appropriate wells. Plates were incubated for 72 hours and cell number was determined using a CellTiter Glo kit according to the manufacturer's instructions (Promega) to generate a measurable luminescent readout proportional to levels of ATP and hence to cell number, for each of the conditions tested. All conditions were performed in triplicate.

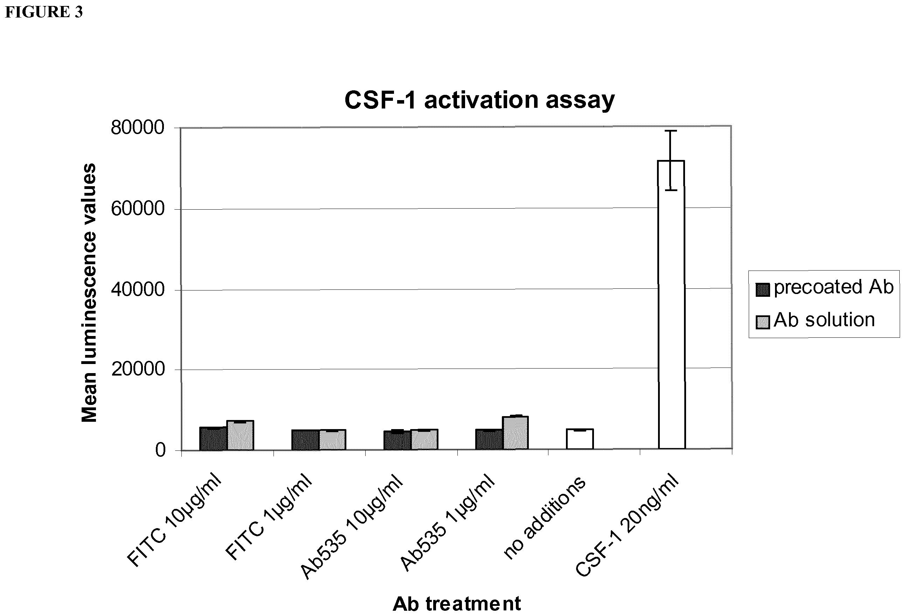

Analysis of the data confirmed that M-NFS-60 are dependent on CSF-1 for proliferation, as reflected by the significantly higher cell number in wells supplemented with CSF-1 compared with those maintained in growth medium without CSF-1. Furthermore, Ab535, either immobilised on the surface of the tissue culture well, or free in solution, did not support the proliferation of M-NFS-60 cells in the absence of CSF-1 at the concentrations used **(****Figure 3****).**

### Example 3. Effect of anti-CSF-lR antibody in a Bleomycin induced in vivo model of pulmonary fibrosis

Bleomycin is an antibiotic first isolated from *Streptomyces verticillatus* and has been used as a chemotherapeutic for various cancers. The bleomycin model of lung fibrosis is a well-established model and was used essentially as described in Madtes, DK et al, 1999, Am J Respir Cell Mol Biol, 20, 924-34. The detailed protocol can be found in the following reference Morschl, E., Molina, J. G., Volmer, J., Mohsenin, A., Pero, R. S., Hong, J. S., Kheradmand, F., Lee, J. J. and Blackburn, M. R. (2008), A3 adenosine receptor signaling influences pulmonary inflammation and fibrosis. Am. J. Respir. Cell Mol. Biol.39: 697-705.
All mice used were wild type C57Blk6 female mice (20g) purchased from Harlan Labs. An intratracheal (IT) cut down instillation was used where mice were anaesthetised with avertin and a tracheostomy performed in order to instil a 3.5 Unit dose of bleomycin in 50 µl of saline or 50 µl of saline alone as a control. Treatment in this manner leads to an inflammatory phase that peaks on day 7 after bleomycin exposure and a fibrotic phase that is maximum on day 21 post exposure. The effect of antibody Ab535 was investigated where antibody was dosed only in the fibrotic phase of the model and was administered subcutaneously at 30mg/kg, three times per week from day 9-21. Animals were sacrificed at day 21 and readouts included histopathological analysis of the lungs, BAL (bronchoalveolar lavage) fluid cellularity and the measurement of soluble collagen in BAL fluid. Histopathological analysis was conducted to score lung damage using a modified Ashcroft scoring system to determine the severity of lung fibrosis (Hubner RH et al, 2008, Biotechniques 44: 507-17). Excised lungs were inflated with 10% formalin to 25cm pressure and processed through a series of alcohols and xylene, embedded in paraffin and tissue sections de-paraffinised prior to processing and staining with Masson's Trichrome.
The amount of soluble collagen in BAL fluid was assessed using a commercially available Sircol assay kit following the manufacturer's instructions.
The effect on the macrophage population in the BAL fluid was determined using cytospin preparations. Aliquots of BAL cells were spun onto microscope slides, stained with Diff-Quick and macrophages counted.

It was found that therapeutic treatment with the anti-CSF-lR antibody, Ab535, with dosing started at day 9 resulted in greatly reduced bleomycin-induced lung fibrosis. Both the severity and extent of fibrosis was significantly reduced. The treated mice had reduced collagen production, improved fibrotic pathology and improved pulmonary barrier protection.

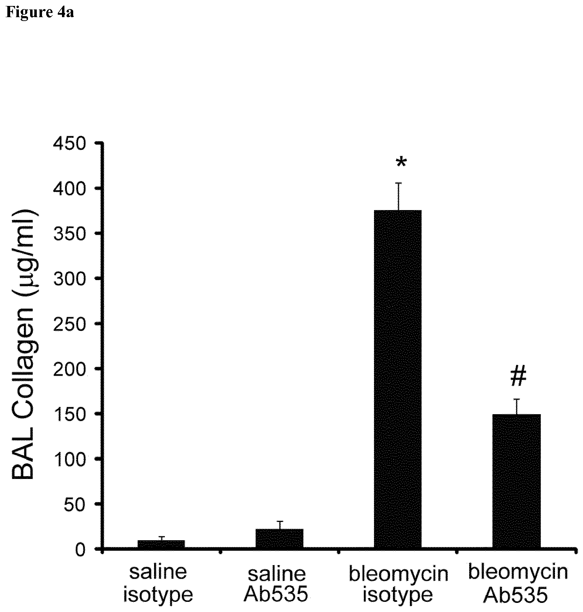

**Figure 4a** shows that treatment of bleomycin-induced lung fibrosis with Ab535 reduced the amount of collagen in BAL fluid compared to treatment with the isotype control.

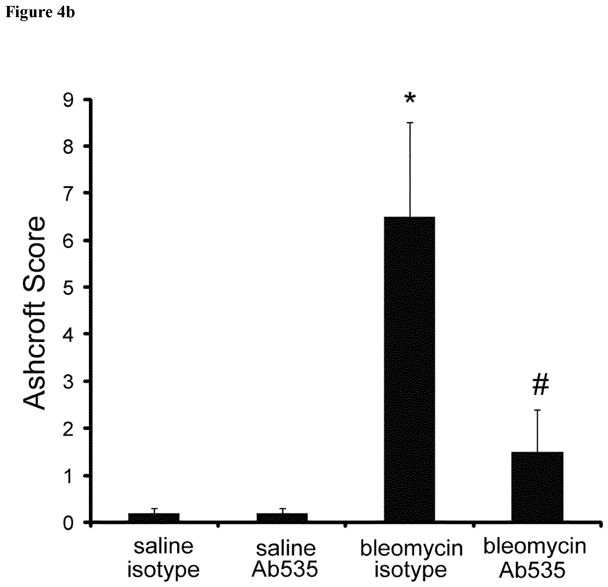

**Figure 4b** shows that treatment of bleomycin-induced lung fibrosis with Ab535 reduced the Ashcroft score of the samples compared to treatment with the isotype control demonstrating that the mice treated with Ab535 had improved fibrotic pathology.

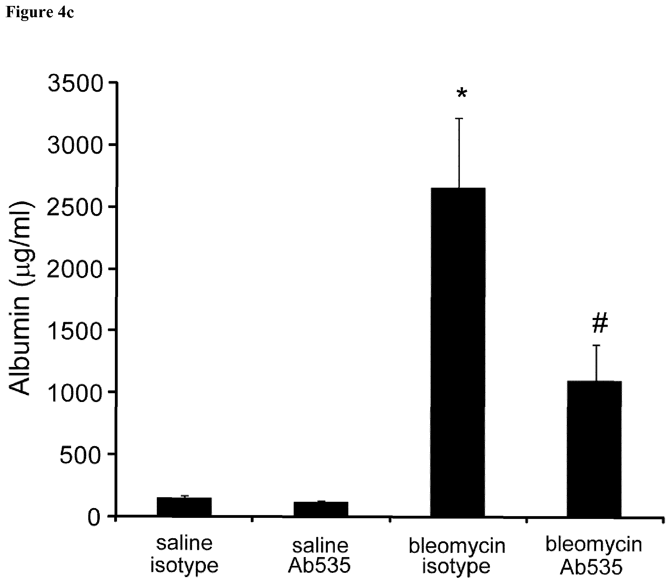

**Figure 4c** shows that treatment of bleomycin-induced lung fibrosis with Ab535 reduced the amount of albumin in BAL fluid compared to treatment with the isotype control demonstrating that the vascular permeability and pulmonary barrier integrity of the mice treated with Ab535 was improved.

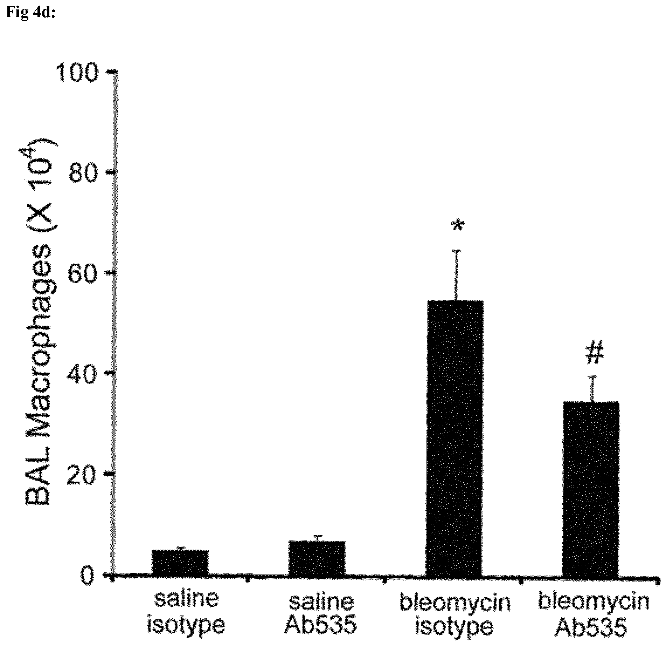

**Figure 4d** shows that treatment of bleomycin-induced lung fibrosis with Ab535 reduced the number of macrophages in the BAL fluid.

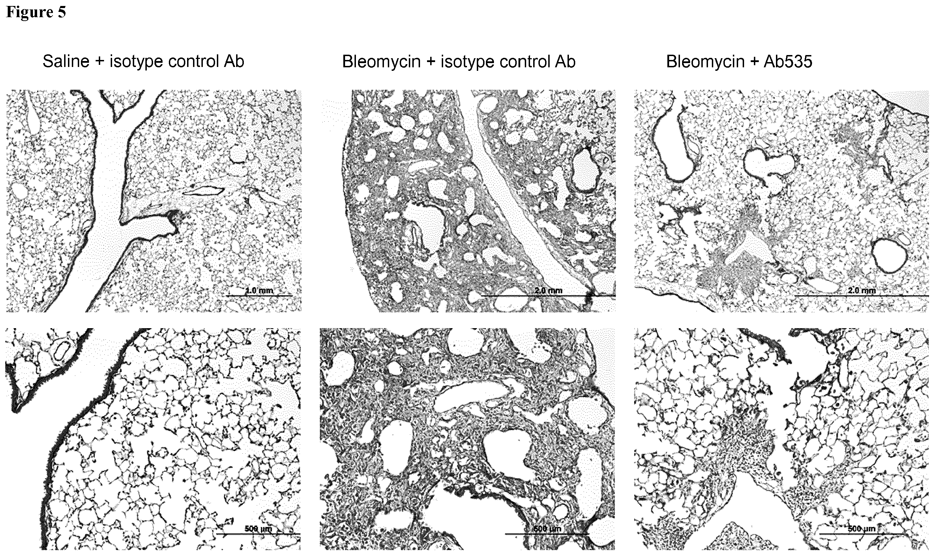

**Figure 5** shows representative images of the histopathological analysis of lungs from saline control, bleomycin plus isotype control and bleomycin plus Ab 535 treated animals. Animals treated with Ab 535 had a greatly reduced fibrosis of the lungs compared to the isotype control, bleomycin treated animals.

### Example 4. Effect of anti-CSF-lR antibody in an adenosine deaminase-deficient mouse model of pulmonary fibrosis

Adenosine is a potent signalling nucleoside, the levels of which increase when cells suffer stress or are damaged and a wide variety of responses are produced when adenosine engages its specific G protein coupled receptors. Adenosine deaminase (ADA) is a purine catabolism enzyme that converts adenosine to inosine. ADA knockout mice have been generated and shown to have increased levels of adenosine in serum as well as in tissues such as kidney, liver and lung (Blackburn, MR et al, 1998, J Biol Chem, 273(9): 5093-5100). These mice exhibit features of chronic lung disease such as alveolar destruction, airway inflammation and excessive mucus production which are associated with increased levels of adenosine in the lung (Blackburn MR et al, 2000, J Exp Med, 192: 159-70). The effects are such that the mice die by three weeks of age due to respiratory distress. Administration of exogenous ADA using a low-dose regimen reduces adenosine levels and extends the life-span of these mice allowing a model of pulmonary fibrosis to be developed (Chunn JL et al, 2005, J Immunol 175: 1937-46, Pedrosa M et al, 2011, PLoS One, 6(7): e22667). In this model, chronic elevation of adenosine levels is associated with an increase in pro-fibrotic mediators including TGFβ-1 in the lungs, increased collagen deposition in lung tissue and increased fibrotic lung pathology. To investigate the effect of molecules with anti-fibrotic potential, ADA-deficient mice are maintained on a low dose exogenous ADA regimen for several weeks, the ADA treatment is then stopped and the potential anti-fibrotic agent administered.

The effect of the anti-CSF-1R antibody Ab535 was investigated in the ADA knockout mouse model of pulmonary fibrosis. In this model, the enzyme deficient mice were maintained on ADA enzyme therapy from day 1 to day 21 post-birth. An ADA-polyethylene glycol (PEG) conjugate was prepared (Young HW et al, 2004, J Immunol 173: 1380-89) and intramuscular injections administered on postnatal days 1, 5, 9, 13 and 17 (0.625, 1.25, 2.5, 2.5 and 2.5 units respectively), followed by 5 units injected intraperitoneally on day 21. No further enzyme was administered after day 21. Ab535 was dosed subcutaneously at 30mg/kg, in a volume of 100 µl three times/week from day 25 (post-natally) until the animals were sacrificed on day 42.

Animals were sacrificed at day 42 and readouts included histopathological analysis of the lungs, BAL fluid cellularity and quantitation of soluble collagen in BAL fluid. Histopathological analysis was conducted to score lung damage using a modified Ashcroft scoring system to determine the severity of lung fibrosis (Hubner et al, 2008, Biotechniques 44: 507-17). Excised lungs were inflated with 10% formalin to 25cm pressure and processed through a series of alcohols and xylene, embedded in paraffin and tissue sections de-paraffinised prior to processing and staining with Masson's Trichrome.

The amount of soluble collagen in BAL fluid was also assessed using a commercially available Sircol assay kit following the manufacturer's instructions.

The effect on the macrophage population in the BAL fluid was determined using cytospin preparations. Aliquots of BAL cells were spun onto microscope slides, stained with Diff-Quick and macrophages counted.

It was found that therapeutic treatment with anti-CSF-lR antibody Ab535 significantly reduced lung fibrosis in ADA-deficient mice. The treated mice had reduced collagen production, improved fibrotic pathology and improved pulmonary barrier function and protection..

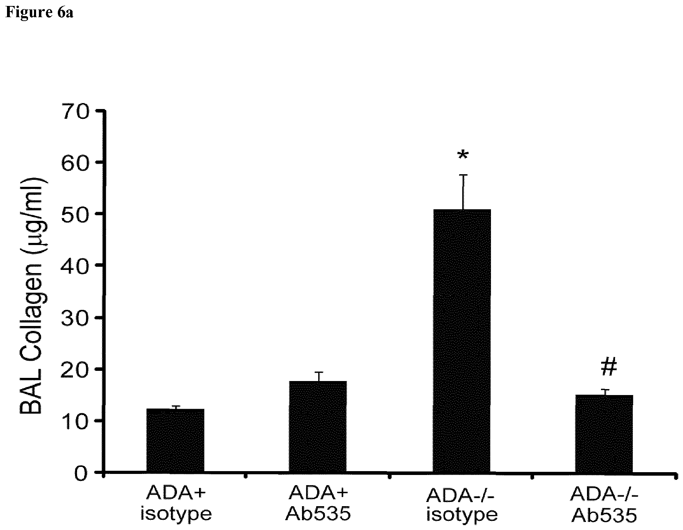

**Figure 6a** shows that treatment of ADA-deficient mice with induced lung fibrosis with Ab535 reduced BAL fluid collagen concentration compared to treatment with the isotype control.

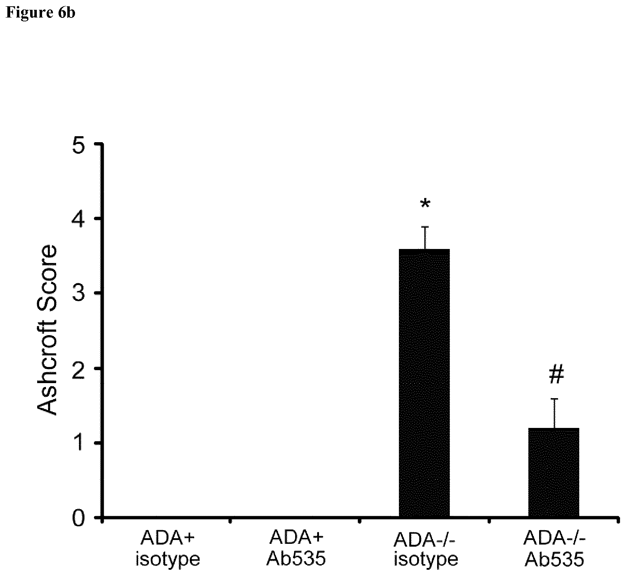

**Figure 6b** shows that treatment of ADA-deficient mice with induced lung fibrosis with Ab535 reduced the Ashcroft score of the samples compared to treatment with the isotype control demonstrating that the mice treated with Ab535 had improved fibrotic pathology.

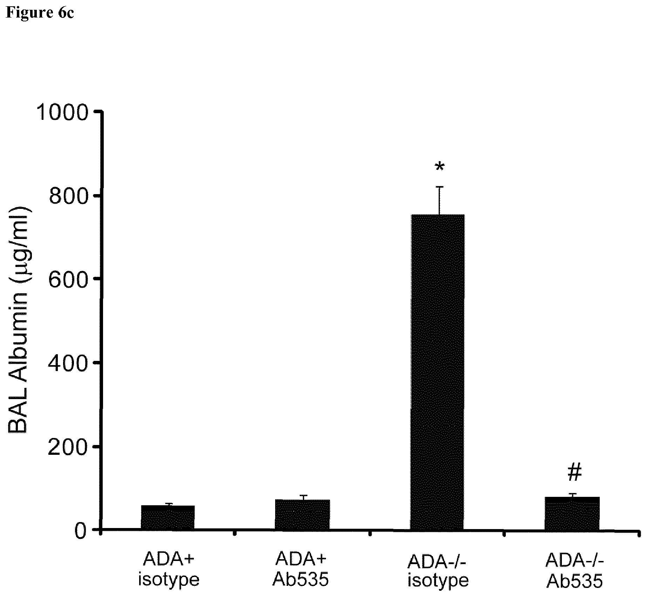

**Figure 6c** shows that treatment of ADA-deficient mice with induced lung fibrosis with Ab535 reduced the concentration of albumin in the BAL fluid compared to treatment with the isotype control demonstratingthat the vascular permeability and pulmonary barrier integrity of the mice treated with Ab535 was improved.

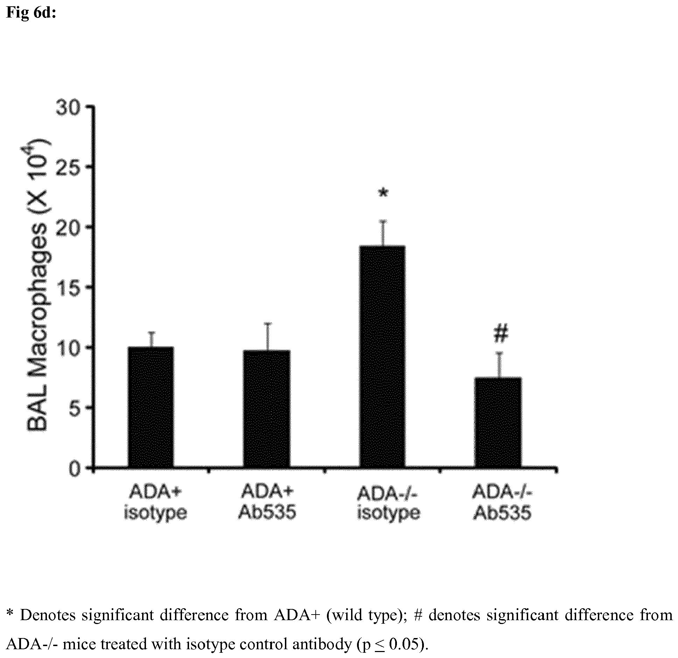

**Figure 6d** shows that treatment of ADA-deficient mice with induced lung fibrosis, with Ab535, reduced the number of macrophages in BAL fluid.

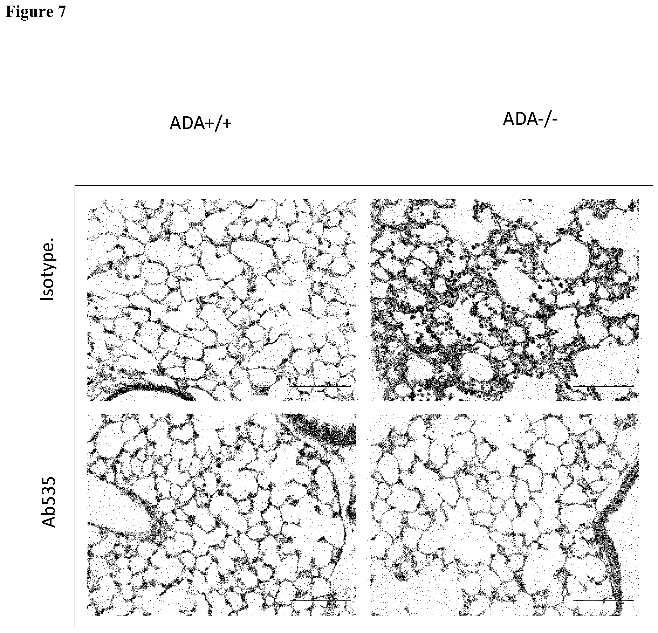

**Figure 7** shows representative images of the histopathological analysis of lung tissue from ADA knockout mice and wild type mice either treated with Ab535 or an isotype control antibody (mouse IgG1). Lung tissue sections were stained with Masson's trichrome. Treatment of ADA knockout mice with Ab535 greatly reduced the amount of fibrotic tissue and collagen deposition.

Accordingly, it has been shown in two mouse models of lung fibrosis that treatment with an anti-CSF-lR antibody can effectively treat fibrotic disease.

## Claims

1. An inhibitor of colony stimulating factor 1 receptor (CSF-1R) activity for use in the treatment and/or prophylaxis of pulmonary fibrosis, wherein the inhibitor is an antibody or functionally active fragment thereof and wherein the antibody or fragment thereof binds to CSF-1R.

2. The inhibitor of CSF-1R activity for the use according to claim 1, wherein the antibody or fragment thereof is monoclonal, polyclonal, chimeric, humanised or bispecific.

3. The inhibitor of CSF-1R activity for the use according to claim 1 or claim 2 wherein the antibody fragment is a Fab, Fab', F(ab')₂, scFv or an epitope-binding fragment thereof.

4. The inhibitor of CSF-1R activity for the use according to any one of claims 1 to 3 wherein the antibody or fragment thereof is conjugated to one or more effector molecule(s).

5. The inhibitor of CSF-1R activity for the use according to any preceding claim, wherein the pulmonary fibrosis is idiopathic pulmonary fibrosis (IPF).

6. The inhibitor of CSF-1R activity for use according to any one of claims 1 to 5 where the inhibitor of CSF-1R activity is administered in combination with one or more other therapeutically active compounds.

7. The inhibitor of CSF-1R activity for use according to claim 6 in which the other therapeutically active compound is another anti-fibrotic therapeutic agent.

## Patentansprüche

1. Inhibitor von CSF-1R(Colony Stimulating Factor 1 Receptor)-Aktivität zur Verwendung bei der Behandlung und/oder Prophylaxe von Lungenfibrose, wobei es sich bei dem Inhibitor um einen Antikörper bzw. ein funktionsfähiges Fragment davon handelt und wobei der Antikörper bzw. das Fragment davon an CSF-1R bindet.

2. Inhibitor von CSF-1R-Aktivität zur Verwendung nach Anspruch 1, wobei der Antikörper bzw. das Fragment davon monoklonal, polyklonal, chimär, humanisiert oder bispezifisch ist.

3. Inhibitor von CSF-1R-Aktivität zur Verwendung nach Anspruch 1 oder Anspruch 2, wobei es sich bei dem Antikörperfragment um ein Fab, Fab', F(ab')₂, scFv oder ein epitopbindendes Fragment davon handelt.

4. Inhibitor von CSF-1R-Aktivität zur Verwendung nach einem der Ansprüche 1 bis 3, wobei der Antikörper bzw. das Fragment davon an ein oder mehrere Effektormolekül(e) konjugiert ist.

5. Inhibitor von CSF-1R-Aktivität zur Verwendung nach einem vorhergehenden Anspruch, wobei es sich bei der Lungenfibrose um idiopathische pulmonale Fibrose (IPF) handelt.

6. Inhibitor von CSF-1R-Aktivität zur Verwendung nach einem der Ansprüche 1 bis 5, wo der Inhibitor von CSF-1R-Aktivität in Kombination mit einer oder mehreren anderen therapeutisch wirksamen Verbindungen verabreicht wird.

7. Inhibitor von CSF-1R-Aktivität zur Verwendung nach Anspruch 6, bei der es sich bei der anderen therapeutisch wirksamen Verbindung um ein weiteres antifibrotisches Therapeutikum handelt.

## Revendications

1. Inhibiteur d'activité du récepteur de facteur stimulant les colonies 1 (CSF-1R) pour utilisation dans le traitement et/ou la prophylaxie de la fibrose pulmonaire, l'inhibiteur étant un anticorps ou un fragment fonctionnellement actif de celui-ci et l'anticorps ou fragment de celui-ci se liant à CSF-1R.

2. Inhibiteur d'activité CSF-1R pour l'utilisation selon la revendication 1, dans lequel l'anticorps ou fragment de celui-ci est monoclonal, polyclonal, chimérique, humanisé ou bispécifique.

3. Inhibiteur d'activité CSF-1R pour l'utilisation selon la revendication 1 ou la revendication 2, dans lequel le fragment d'anticorps est un Fab, Fab', F(ab')₂, scFv ou un fragment de liaison d'épitope de ceux-ci.

4. Inhibiteur d'activité CSF-1R pour l'utilisation selon l'une quelconque des revendications 1 à 3, dans lequel l'anticorps ou fragment de celui-ci est conjugué à une ou plusieurs molécule(s) effectrice(s).

5. Inhibiteur d'activité CSF-1R pour l'utilisation selon l'une quelconque des revendications précédentes, la fibrose pulmonaire étant la fibrose pulmonaire idiopathique (FPI).

6. Inhibiteur d'activité CSF-1R pour utilisation selon l'une quelconque des revendications 1 à 5, l'inhibiteur d'activité CSF-1R étant administré en combinaison avec un ou plusieurs autres composés thérapeutiquement actifs.

7. Inhibiteur d'activité CSF-1R pour utilisation selon la revendication 6, l'autre composé thérapeutiquement actif étant un autre agent thérapeutique antifibrotique.


### SEQUENCE LISTING

<110> UCB Biopharma SPRL
<120> METHOD FOR THE TREATMENT OF FIBROTIC DISEASE
<130> G0180-PCT
<150> GB1315486.9
   <151> 2013-08-30
<160> 5
<170> PatentIn version 3.5
<210> 1
   <211> 972
   <212> PRT
   <213> Artificial Sequence
<220>
   <223> Human CSF-1R sequence
<400> 1
<210> 2
   <211> 277
   <212> PRT
   <213> Artificial Sequence
<220>
   <223> Amino acid sequence for CSF-1R
<400> 2
   Ile Pro Val Ile Glu Pro Ser Val Pro Glu Leu Val Val Lys Pro Gly
   1               5                   10                  15
   Ala Thr Val Thr Leu Arg Cys Val Gly Asn Gly Ser Val Glu Trp Asp
               20                  25                  30
   Gly Pro Pro Ser Pro His Trp Thr Leu Tyr Ser Asp Gly Ser Ser Ser
           35                  40                  45
   Ile Leu Ser Thr Asn Asn Ala Thr Phe Gln Asn Thr Gly Thr Tyr Arg
       50                  55                  60
   Cys Thr Glu Pro Gly Asp Pro Leu Gly Gly Ser Ala Ala Ile His Leu
   65                  70                  75                  80
   Tyr Val Lys Asp Pro Ala Arg Pro Trp Asn Val Leu Ala Gln Glu Val
                   85                  90                  95
   Val Val Phe Glu Asp Gln Asp Ala Leu Leu Pro Cys Leu Leu Thr Asp
               100                 105                 110
   Pro Val Leu Glu Ala Gly Val Ser Leu Val Arg Val Arg Gly Arg Pro
           115                 120                 125
   Leu Met Arg His Thr Asn Tyr Ser Phe Ser Pro Trp His Gly Phe Thr
       130                 135                 140
   Ile His Arg Ala Lys Phe Ile Gln Ser Gln Asp Tyr Gln Cys Ser Ala
   145                 150                 155                 160
   Leu Met Gly Gly Arg Lys Val Met Ser Ile Ser Ile Arg Leu Lys Val
                   165                 170                 175
   Gln Lys Val Ile Pro Gly Pro Pro Ala Leu Thr Leu Val Pro Ala Glu
               180                 185                 190
   Leu Val Arg Ile Arg Gly Glu Ala Ala Gln Ile Val Cys Ser Ala Ser
           195                 200                 205
   Ser Val Asp Val Asn Phe Asp Val Phe Leu Gln His Asn Asn Thr Lys
       210                 215                 220
   Leu Ala Ile Pro Gln Gln Ser Asp Phe His Asn Asn Arg Tyr Gln Lys
   225                 230                 235                 240
   Val Leu Thr Leu Asn Leu Asp Gln Val Asp Phe Gln His Ala Gly Asn
                   245                 250                 255
   Tyr Ser Cys Val Ala Ser Asn Val Gln Gly Lys His Ser Thr Ser Met
               260                 265                 270
   Phe Phe Arg Val Val
           275
<210> 3
   <211> 49
   <212> PRT
   <213> Artificial Sequence
<220>
   <223> Amino acid sequence for CSF-1R (ENSP00000421174 49 amino acids)
<400> 3
   Met Arg His Thr Asn Tyr Ser Phe Ser Pro Trp His Gly Phe Thr Ile
   1               5                   10                  15
   His Arg Ala Lys Phe Ile Gln Ser Gln Asp Tyr Gln Cys Ser Ala Leu
               20                  25                  30
   Met Gly Gly Arg Lys Val Met Ser Ile Ser Ile Arg Leu Lys Val Gln
           35                  40                  45
   Lys
<210> 4
   <211> 493
   <212> PRT
   <213> Artificial Sequence
<220>
   <223> Amino acid sequence for CSF-1R (SNP V32G, A245S, H247P, V279M, position underlined)
<400> 4
   Ile Pro Val Ile Glu Pro Ser Val Pro Glu Leu Val Val Lys Pro Gly
   1               5                   10                  15
   Ala Thr Val Thr Leu Arg Cys Val Gly Asn Gly Ser Val Glu Trp Asp
               20                  25                  30
   Gly Pro Pro Ser Pro His Trp Thr Leu Tyr Ser Asp Gly Ser Ser Ser
           35                  40                  45
   Ile Leu Ser Thr Asn Asn Ala Thr Phe Gln Asn Thr Gly Thr Tyr Arg
       50                  55                  60
   Cys Thr Glu Pro Gly Asp Pro Leu Gly Gly Ser Ala Ala Ile His Leu
   65                  70                  75                  80
   Tyr Val Lys Asp Pro Ala Arg Pro Trp Asn Val Leu Ala Gln Glu Val
                   85                  90                  95
   Val Val Phe Glu Asp Gln Asp Ala Leu Leu Pro Cys Leu Leu Thr Asp
               100                 105                 110
   Pro Val Leu Glu Ala Gly Val Ser Leu Val Arg Val Arg Gly Arg Pro
           115                 120                 125
   Leu Met Arg His Thr Asn Tyr Ser Phe Ser Pro Trp His Gly Phe Thr
       130                 135                 140
   Ile His Arg Ala Lys Phe Ile Gln Ser Gln Asp Tyr Gln Cys Ser Ala
   145                 150                 155                 160
   Leu Met Gly Gly Arg Lys Val Met Ser Ile Ser Ile Arg Leu Lys Val
                   165                 170                 175
   Gln Lys Val Ile Pro Gly Pro Pro Ala Leu Thr Leu Val Pro Ala Glu
               180                 185                 190
   Leu Val Arg Ile Arg Gly Glu Ala Ala Gln Ile Val Cys Ser Ala Ser
           195                 200                 205
   Ser Val Asp Val Asn Phe Asp Val Phe Leu Gln His Asn Asn Thr Lys
               210                 215                 220
   Leu Ala Ile His Gln Gln Ser Asp Phe His Asn Asn Arg Tyr Gln Lys
   225                 230                 235                 240
   Val Leu Thr Leu Asn Leu Asp Gln Val Asp Phe Gln His Ala Gly Asn
                   245                 250                 255
   Tyr Ser Cys Val Ala Ser Asn Val Gln Gly Lys His Ser Thr Ser Met
               260                 265                 270
   Phe Phe Arg Val Val Glu Ser Ala Tyr Leu Asn Leu Ser Ser Glu Gln
           275                 280                 285
   Asn Leu Ile Gln Glu Val Thr Val Gly Glu Gly Leu Asn Leu Lys Val
       290                 295                 300
   Met Val Glu Ala Tyr Pro Gly Leu Gln Gly Phe Asn Trp Thr Tyr Leu
   305                 310                 315                 320
   Gly Pro Phe Ser Asp His Gln Pro Glu Pro Lys Leu Ala Asn Ala Thr
                   325                 330                 335
   Thr Lys Asp Thr Tyr Arg His Thr Phe Thr Leu Ser Leu Pro Arg Leu
               340                 345                 350
   Lys Pro Ser Glu Ala Gly Arg Tyr Ser Phe Leu Ala Arg Asn Pro Gly
           355                 360                 365
   Gly Trp Arg Ala Leu Thr Phe Glu Leu Thr Leu Arg Tyr Pro Pro Glu
       370                 375                 380
   Val Ser Val Ile Trp Thr Phe Ile Asn Gly Ser Gly Thr Leu Leu Cys
   385                 390                 395                 400
   Ala Ala Ser Gly Tyr Pro Gln Pro Asn Val Thr Trp Leu Gln Cys Ser
                   405                 410                 415
   Gly His Thr Asp Arg Cys Asp Glu Ala Gln Val Leu Gln Val Trp Asp
               420                 425                 430
   Asp Pro Tyr Pro Glu Val Leu Ser Gln Glu Pro Phe His Lys Val Thr
           435                 440                 445
   Val Gln Ser Leu Leu Thr Val Glu Thr Leu Glu His Asn Gln Thr Tyr
       450                 455                 460
   Glu Cys Arg Ala His Asn Ser Val Gly Ser Gly Ser Trp Ala Phe Ile
   465 470 475 480
   Pro Ile Ser Ala Gly Ala His Thr His Pro Pro Asp Glu
   485 490
<210> 5
   <211> 512
   <212> PRT
   <213> Artificial Sequence
<220>
   <223> CSF-1R form suitable for expression on a cell surface
<400> 5
   Met Gly Pro Gly Val Leu Leu Leu Leu Leu Val Ala Thr Ala Trp His
   1               5                   10                  15
   Gly Gln Gly Ile Pro Val Ile Glu Pro Ser Val Pro Glu Leu Val Val
               20                  25                  30
   Lys Pro Gly Ala Thr Val Thr Leu Arg Cys Val Gly Asn Gly Ser Val
           35                  40                  45
   Glu Trp Asp Gly Pro Pro Ser Pro His Trp Thr Leu Tyr Ser Asp Gly
       50                  55                  60
   Ser Ser Ser Ile Leu Ser Thr Asn Asn Ala Thr Phe Gln Asn Thr Gly
   65                  70                  75                  80
   Thr Tyr Arg Cys Thr Glu Pro Gly Asp Pro Leu Gly Gly Ser Ala Ala
                   85                  90                  95
   Ile His Leu Tyr Val Lys Asp Pro Ala Arg Pro Trp Asn Val Leu Ala
               100                 105                 110
   Gln Glu Val Val Val Phe Glu Asp Gln Asp Ala Leu Leu Pro Cys Leu
           115                 120                 125
   Leu Thr Asp Pro Val Leu Glu Ala Gly Val Ser Leu Val Arg Val Arg
       130                 135                 140
   Gly Arg Pro Leu Met Arg His Thr Asn Tyr Ser Phe Ser Pro Trp His
   145                 150                 155                 160
   Gly Phe Thr Ile His Arg Ala Lys Phe Ile Gln Ser Gln Asp Tyr Gln
                   165                 170                 175
   Cys Ser Ala Leu Met Gly Gly Arg Lys Val Met Ser Ile Ser Ile Arg
               180                 185                 190
   Leu Lys Val Gln Lys Val Ile Pro Gly Pro Pro Ala Leu Thr Leu Val
           195                 200                 205
   Pro Ala Glu Leu Val Arg Ile Arg Gly Glu Ala Ala Gln Ile Val Cys
       210                 215                 220
   Ser Ala Ser Ser Val Asp Val Asn Phe Asp Val Phe Leu Gln His Asn
   225                 230                 235                 240
   Asn Thr Lys Leu Ala Ile Pro Gln Gln Ser Asp Phe His Asn Asn Arg
                   245                 250                 255
   Tyr Gln Lys Val Leu Thr Leu Asn Leu Asp Gln Val Asp Phe Gln His
               260                 265                 270
   Ala Gly Asn Tyr Ser Cys Val Ala Ser Asn Val Gln Gly Lys His Ser
           275                 280                 285
   Thr Ser Met Phe Phe Arg Val Val Glu Ser Ala Tyr Leu Asn Leu Ser
       290                 295                 300
   Ser Glu Gln Asn Leu Ile Gln Glu Val Thr Val Gly Glu Gly Leu Asn
   305                 310                 315                 320
   Leu Lys Val Met Val Glu Ala Tyr Pro Gly Leu Gln Gly Phe Asn Trp
                   325                 330                 335
   Thr Tyr Leu Gly Pro Phe Ser Asp His Gln Pro Glu Pro Lys Leu Ala
               340                 345                 350
   Asn Ala Thr Thr Lys Asp Thr Tyr Arg His Thr Phe Thr Leu Ser Leu
           355                 360                 365
   Pro Arg Leu Lys Pro Ser Glu Ala Gly Arg Tyr Ser Phe Leu Ala Arg
       370                 375                 380
   Asn Pro Gly Gly Trp Arg Ala Leu Thr Phe Glu Leu Thr Leu Arg Tyr
   385                 390                 395                 400
   Pro Pro Glu Val Ser Val Ile Trp Thr Phe Ile Asn Gly Ser Gly Thr
                   405                 410                 415
   Leu Leu Cys Ala Ala Ser Gly Tyr Pro Gln Pro Asn Val Thr Trp Leu
               420                 425                 430
   Gln Cys Ser Gly His Thr Asp Arg Cys Asp Glu Ala Gln Val Leu Gln
           435                 440                 445
   Val Trp Asp Asp Pro Tyr Pro Glu Val Leu Ser Gln Glu Pro Phe His
       450                 455                 460
   Lys Val Thr Val Gln Ser Leu Leu Thr Val Glu Thr Leu Glu His Asn
   465                 470                 475                 480
   Gln Thr Tyr Glu Cys Arg Ala His Asn Ser Val Gly Ser Gly Ser Trp
                   485                 490                 495
   Ala Phe Ile Pro Ile Ser Ala Gly Ala His Thr His Pro Pro Asp Glu
               500                 505                 510